# EUROPEAN PATENT SPECIFICATION

(11) **EP 3 341 448 B1**
(45) Date of publication and mention of the grant of the patent: **12.02.2020**
(21) Application number: 16745626.8
(22) Date of filing: 29.07.2016
(51) Int. Cl.: C09K 11/06, C07C 15/30, C07D 307/00, C07D 307/02, H01L 51/00, C07C 255/50, C07C 43/275, C07B 59/00, C07D 235/18, C07D 239/26, C07D 251/24, C07D 213/16, C07D 307/91, H01L 51/50

(54) **COMPOUNDS FOR ELECTRONIC DEVICES**
VERBINDUNGEN FÜR ELEKTRONISCHE VORRICHTUNGEN
COMPOSÉS POUR DISPOSITIFS ÉLECTRONIQUES

(30) Priority: 28.08.2015 EP 15182962
(43) Date of publication of application: 04.07.2018
(73) Proprietor: Merck Patent GmbH, 64293 Darmstadt (DE)
(72) Inventor: RODRIGUEZ, Lara-Isabel, 64283 Darmstadt (DE); LINGE, Rouven, 64291 Darmstadt (DE); MEYER, Sebastian, 63741 Aschaffenburg (DE); HEIL, Holger, 60316 Frankfurt am Main (DE)
(86) International application number: PCT/EP2016/001319
(87) International publication number: WO 2017/036573

(56) References cited:
- WO-A1-2014/106522
- WO-A2-2014/111269
- DE-A1-102008 008 953
- DE-A1-102009 005 746
- KR-A- 20150 084 562

## Description

The present application relates to phenanthrene compounds of a particular structure further defined below, and to the use of such compounds in electronic devices, in particular in organic electroluminescent devices (OLEDs). Further, the application relates to particular embodiments of electronic devices, comprising the phenanthrene compounds, and to processes for the synthesis of the phenanthrene compounds.

In accordance with the present invention, the term electronic device is taken to mean in general electronic devices which comprise organic materials. These are preferably taken to mean OLEDs and some further embodiments of electronic devices comprising organic materials which are disclosed later in the application.

The general structure and principle of functioning of OLEDs are known to the person skilled in the art and are described, inter alia, in US 4539507, US 5151629, EP 0676461 and WO 1998/27136.

With respect to the performance data of the electronic devices, further improvements are necessary, in particular with a view to broad commercial use, for example in displays or as light sources. Of particular importance in this connection are the lifetime, the efficiency and the operating voltage of the electronic devices and the colour values achieved.

In particular in the case of blue-emitting OLEDs, there is potential for improvement with respect to the lifetime of the devices, the efficiency and the colour coordinates of the emitted light.

An important starting point for achieving these improvements is the choice of the matrix material which is employed in the emitting layer of the OLED. Here, the term matrix material is understood to mean a material which does not emit light during operation of the OLED, and which is present in the emitting layer of an OLED together with at least one emitting material.

In the prior art, it is known that 3-phenanthrenyl anthracene compounds, such as disclosed in WO 2009/100925, are well suitable for use as matrix materials in OLEDs. Compounds comprising phenanthrenyl anthracene substituted with phenyl groups, as disclosed in KR20150084562, are also known from the prior art,

However, there continues to be a need for matrix materials which show excellent performance data when used in the emitting layer of an OLED, in particular in regard to lifetime, efficiency and colour values of the emitted light.

Further, there exists a need for novel electron transporting materials, in particular for use in an electron transporting layer of an OLED.

Now, unexpectedly, it has been found that phenanthrene compounds which have an aryl or heteroaryl group bonded to the 9-position of a phenenthrene, which is in turn bonded in its 3-position to a 9,10-anthracenylene moiety, which has a particular aryl or heteroaryl group bonded to the other side of the anthracenylene moiety, show very favorable properties, in particular when used as matrix materials or as electron transporting materials, in respect to lifetime, efficiency and color coordinates of the emitted light.

Object of the present invention is thus a compound of a formula (I) 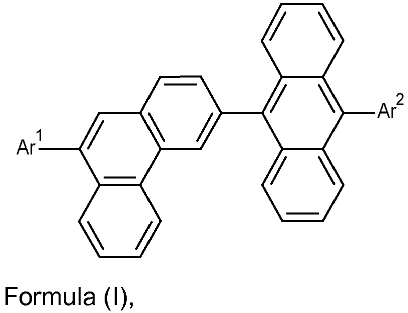 where the phenanthrene group may be substituted by radicals R³ in the free positions, and the anthracene group may be substituted by radicals R⁴ in the free positions, and
where the following applies to the variables occuring:
- Ar¹: is selected from aryl groups having 6 to 10 aromatic ring atoms, which may be substituted by radicals R¹, or heteroaryl groups having 5 to 18 aromatic ring atoms, which may be substituted by radicals R¹;
- Ar²: is selected from 1-naphthyl, which may be substituted by radicals R², or aromatic ring systems having 13 to 30 aromatic ring atoms, which may be substituted by radicals R², or heteroaromatic ring systems having 5 to 30 aromatic ring atoms, which may be substituted by radicals R²;
- R¹, R², R⁴: is selected, identically or differently at each occurrence, from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, or heteroaromatic ring systems having 5 to 40 aromatic ring atoms; where two or more radicals R¹, R² or R⁴ may be connected to each other to form a ring; where the said alkyl, alkoxy, alkenyl and alkynyl groups and the said aromatic and heteroaromatic ring systems may in each case be substituted by one or more radicals R⁵, and where one or more CH₂ groups in the said alkyl, alkoxy, alkenyl and alkynyl groups may in each case be replaced by -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO or SO₂;
- R³: is selected, identically or differently on each occurrence, from H, D, F, CN, straight-chain alkyl or alkoxy groups having 1 to 20 C atoms, branched or cyclic alkyl or alkoxy groups having 3 to 20 C atoms, or alkenyl or alkynyl groups having 2 to 20 C atoms, where two or more radicals R³ may be connected to each other to form a ring; where the said alkyl, alkoxy, alkenyl and alkynyl groups may in each case be substituted by one or more radicals R⁵, and where one or more CH₂ groups in the said alkyl, alkoxy, alkenyl and alkynyl groups may in each case be replaced by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂;
- R⁵: is selected, identically or differently at each occurrence, from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, or heteroaromatic ring systems having 5 to 40 aromatic ring atoms; where the said alkyl, alkoxy, alkenyl and alkynyl groups and the said aromatic and heteroaromatic ring systems may in each case be substituted by one or more radicals R⁶, and where one or more CH₂ groups in the said alkyl, alkoxy, alkenyl and alkynyl groups may in each case be replaced by -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO or SO₂;
- R⁶: is selected, identically or differently at each occurrence, from H, D, F, CN, alkyl groups having 1 to 20 C atoms, aromatic ring systems having 6 to 40 C atoms, or heteroaromatic ring systems having 5 to 40 aromatic ring atoms, where the said alkyl groups, aromatic ring systems and heteroaromatic ring systems may be substituted by F and CN.

The following definitions apply to the chemical groups as general definitions. They only apply insofar as no more specific definitions are given.

An aryl group in the sense of this invention contains 6 to 40 aromatic ring atoms, of which none is a heteroatom. An aryl group here is taken to mean either a simple aromatic ring, for example benzene, or a condensed aromatic polycycle, for example naphthalene, phenanthrene, or anthracene. A condensed aromatic polycycle in the sense of the present application consists of two or more simple aromatic rings condensed with one another.

A heteroaryl group in the sense of this invention contains 5 to 40 aromatic ring atoms, at least one of which is a heteroatom. The heteroatoms are preferably selected from N, O and S. A heteroaryl group here is taken to mean either a simple heteroaromatic ring, such as pyridine, pyrimidine or thiophene, or a condensed heteroaromatic polycycle, such as quinoline or carbazole. A condensed heteroaromatic polycycle in the sense of the present application consists of two or more simple heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, pheno-thiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aromatic ring system in the sense of this invention contains 6 to 40 C atoms in the ring system and does not comprise any heteroatoms as aromatic ring atoms. An aromatic ring system in the sense of this application therefore does not comprise any heteroaryl groups. An aromatic ring system in the sense of this invention is intended to be taken to mean a system which does not necessarily contain only aryl groups, but instead in which, in addition, a plurality of aryl groups may be connected by a non-aromatic unit such as one or more optionally substituted C, Si, N, O or S atoms. The non-aromatic unit in such case comprises preferably less than 10% of the atoms other than H, relative to the total number of atoms other than H of the whole aromatic ring system. Thus, for example, systems such as 9,9'-spirobifluorene, 9,9'-diarylfluorene, triarylamine, diaryl ether, and stilbene are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl groups are linked to one another via single bonds are also taken to be aromatic ring systems in the sense of this invention, such as, for example, systems such as biphenyl and terphenyl.

A heteroaromatic ring system in the sense of this invention contains 5 to 40 aromatic ring atoms, at least one of which is a heteroatom. The heteroatoms are preferably selected from N, O or S. A heteroaromatic ring system is defined as an aromatic ring system above, with the difference that it must obtain at least one heteroatom as one of the aromatic ring atoms. It thereby differs from an aromatic ring system according to the definition of the present application, which cannot comprise any heteroatom as aromatic ring atom.

An aromatic ring system having 6 to 40 aromatic ring atoms or a heteroaromatic ring system having 5 to 40 aromatic ring atoms is in particular a group which is derived from the above mentioned aryl or heteroaryl groups, or from biphenyl, terphenyl, quaterphenyl, fluorene, spirobifluorene, dihydrophenanthrene, dihydropyrene, tetrahydropyrene, indenofluorene, truxene, isotruxene, spirotruxene, spiroisotruxene, and indenocarbazole.

For the purposes of the present invention, a straight-chain alkyl group having 1 to 20 C atoms or a branched or cyclic alkyl group having 3 to 20 C atoms or an alkenyl or alkynyl group having 2 to 20 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl.

An alkoxy or thioalkyl group having 1 to 20 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

Regarding ring formation, which is allowed for groups R¹ to R⁴ to occur between each other, as stated above, it is preferred that in this case, two or more radicals R¹ to R⁴ form a condensed aliphatic five-ring or six-ring, such as in the case of two alkyl groups on a phenyl group forming a ring to result in an indane, in particular a hexamethylindane; or two radicals R² or R⁴ which are bonded to one and the same atom form a ring, such as in the case of two radicals in 9- and 9'-position on a fluorene forming a ring and thereby a spiro group.

Group Ar¹ is preferably a phenyl or a naphthyl group, which may each be substituted by one or more radicals R¹.

In a preferred embodiment of the present invention, radicals R¹ are selected, identically or differently on each occurrence, from H, D, F, CN, Si(R⁵)₃, straight-chain alkyl or alkoxy groups having 1 to 20 C atoms, branched or cyclic alkyl or alkoxy groups having 3 to 20 C atoms, aromatic ring systems having 6 to 40 aromatic ring atoms, or heteroaromatic ring systems having 5 to 40 aromatic ring atoms; where the said alkyl and alkoxy groups or aromatic or heteroaromatic ring systems may in each case be substituted by one or more radicals R⁵, and where one or more CH₂ groups in the said alkyl and alkoxy groups may in each case be replaced by -R⁵C=CR⁵-, -C≡C-, Si(R⁵)₂, C=O,
C=NR⁵, -C(=O)O-, -C(=O)NR⁵-, NR⁵, P(=O)(R⁵), -O-, -S-, SO or SO₂. Two or more radicals R¹ may be connected to each other and form a ring. In this case, preferably, a condensed aliphatic five-ring or six-ring is formed, such as in the case of two alkyl groups on a phenyl group forming a ring to result in an indane, in particular a hexamethylindane; or two radicals R¹ which are bonded to one and the same atom form a ring, such as in the case of two radicals in 9- and 9'-position on a fluorene forming a ring and thereby a spiro group.

Particularly preferably, radicals R¹ are selected, identically or differently on each occurrence, from H, D, F, CN, Si(R⁵)₃, straight-chain alkyl or alkoxy groups having 1 to 20 C atoms, or branched or cyclic alkyl or alkoxy groups having 3 to 20 C atoms; where the said alkyl and alkoxy groups may in each case be substituted by one or more radicals R⁵.

Preferred aromatic ring systems as groups Ar² are selected 1-naphthyl, fluorenyl, benzofluorenyl, indenofluorenyl, spirobifluorenyl, benzoindenofluorenyl, phenanthrenyl, anthracenyl, pyrenyl, benzanthracenyl, and benzophenanthrenyl, each optionally substituted by one or more radicals R². Particularly preferred aromatic ring systems as groups Ar² are selected from fluorene, benzofluorene, indenofluorene, spirobifluorene and benzoindenofluorene, each optionally substituted by one or more radicals R².

Group Ar² is preferably a heteroaromatic ring system having 5 to 18 aromatic ring atoms, which may be substituted by radicals R². Preferred heteroaromatic ring systems as groups Ar² are selected from furanyl, benzofuranyl, isobenzofuranyl, dibenzofuranyl, thiophenyl, benzothiophenyl, isobenzothiophenyl, dibenzothiophenyl, indolyl, isoindolyl, carbazolyl, indolocarbazolyl, indenocarbazolyl, pyridyl, quinolinyl, isochinolinyl, acridyl, phenanthridyl, benzimidazolyl, pyrimidyl, pyrazinyl, pyridazinyl, and triazinyl, each optionally substituted by radicals R².

In an alternative, equally preferred embodiment, Ar² is 1-naphthyl, which may be substituted by radicals R².
Preferably, radicals R² and R⁴ are selected, identically or differently on each occurrence, from H, D, F, CN, Si(R⁵)₃, straight-chain alkyl or alkoxy groups having 1 to 20 C atoms, branched or cyclic alkyl or alkoxy groups having 3 to 20 C atoms, aromatic ring systems having 6 to 40 aromatic ring atoms, or heteroaromatic ring systems having 5 to 40 aromatic ring atoms; where the said alkyl and alkoxy groups or aromatic or heteroaromatic ring systems may in each case be substituted by one or more radicals R⁵, and where one or more CH₂ groups in the said alkyl and alkoxy groups may in each case be replaced by -R⁵C=CR⁵-, -C≡C-, Si(R⁵)₂, C=O,
C=NR⁵, -C(=O)O-, -C(=O)NR⁵-, NR⁵, P(=O)(R⁵), -O-, -S-, SO or SO₂. Two or more radicals R² or R⁴ may be connected to each other and form a ring. In this case, preferably, a condensed aliphatic five-ring or six-ring is formed, such as in the case of two alkyl groups on a phenyl group forming a ring to result in an indane, in particular a hexamethylindane; or two radicals R² or R⁴ which are bonded to one and the same atom form a ring, such as in the case of two radicals in 9- and 9'-position on a fluorene forming a ring and thereby a spiro group.

Radicals R³ are preferably selected, identically or differently on each occurrence, from H, D, F, CN, straight-chain alkyl or alkoxy groups having 1 to 20 C atoms, or branched or cyclic alkyl or alkoxy groups having 3 to 20 C atoms, where the said alkyl or alkoxy groups may in each case be substituted by one or more radicals R⁵. More preferably, radicals R³ are selected from H and D. Particularly preferably, the phenanthrene group in formula (I) does not have any substituents R³.

Radicals R⁵ are preferably selected, identically or differently on each occurrence, from H, D, F, CN, Si(R⁶)₃, straight-chain alkyl or alkoxy groups having 1 to 20 C atoms, branched or cyclic alkyl or alkoxy groups having 3 to 20 C atoms, aromatic ring systems having 6 to 40 aromatic ring atoms, or heteroaromatic ring systems having 5 to 40 aromatic ring atoms; where the said alkyl or alkoxy groups and the said aromatic or heteroaromatic ring systems may in each case be substituted by one or more radicals R⁶.

Preferred embodiments of compounds according to formula (I) correspond to the following formula (I-2) 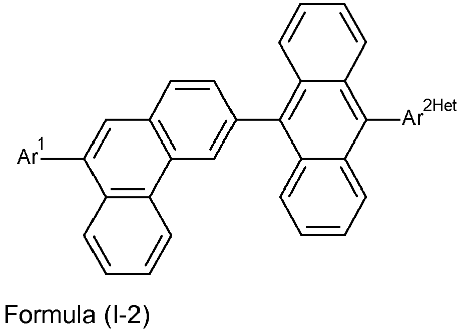 where the phenanthrene group may be substituted by radicals R³ in the free positions, and the anthracene group may be substituted by radicals R⁴ in the free positions, and
where the variables occurring are defined as above, and:
Ar^{2Het} is a heteroaromatic ring system having 5 to 18 aromatic ring atoms, which may be substituted by radicals R².

Compounds of formula (I-2) are particularly suitable as electron transporting compounds in OLEDs.

Preferably, Ar^{2Het} is selected from furanyl, benzofuranyl, isobenzofuranyl, dibenzofuranyl, thiophenyl, benzothiophenyl, isobenzothiophenyl, dibenzothiophenyl, indolyl, isoindolyl, carbazolyl, indolocarbazolyl, indenocarbazolyl, pyridyl, quinolinyl, isochinolinyl, acridyl, phenanthridyl, benzimidazolyl, pyrimidyl, pyrazinyl, pyridazinyl, and triazinyl, which may each be substituted by radicals R².

Furthermore, preferably, in formula (1-2), the phenanthryl group does not have any substituents R³.

Particularly preferably, in formula (I-2), group Ar¹ is selected from phenyl and naphthyl, which may each be substituted by radicals R¹.

Furthermore, all preferred embodiments of variable groups which are listed above in connection with formula (I) apply equally to (I-2).

The following compounds are examples of compounds according to formula (I):

| | | |
|---|---|---|
| 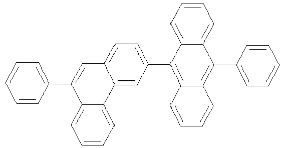 | 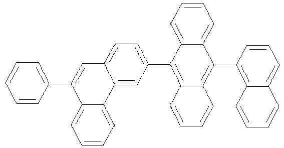 | 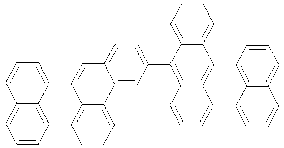 |
| (1)* | (2) | (3) |
| 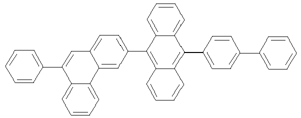 | 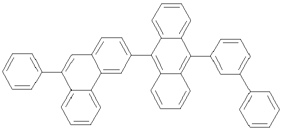 | 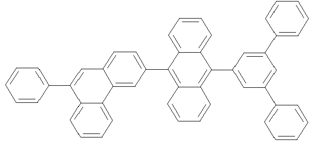 |
| (4) | (5) | (6) |
| 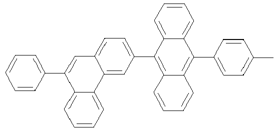 | 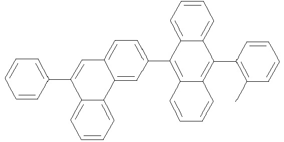 | 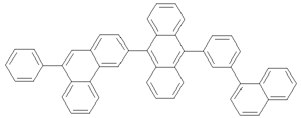 |
| (7)* | (8)* | (9) |
| 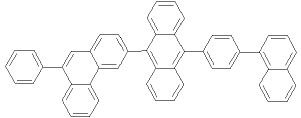 | 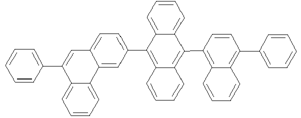 | 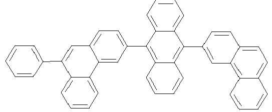 |
| (10) | (11) | (12) |
| 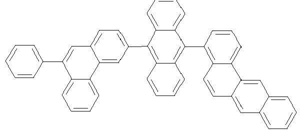 | 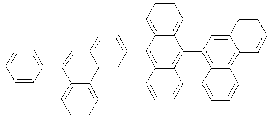 | 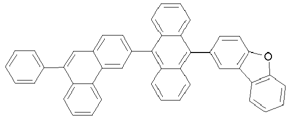 |
| (13) | (14) | (15) |
| 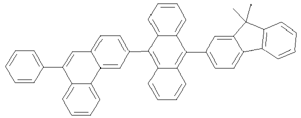 | 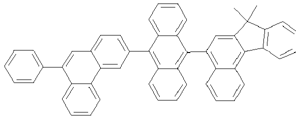 | 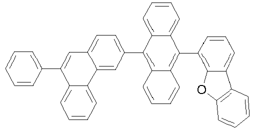 |
| (16) | (17) | (18) |
| 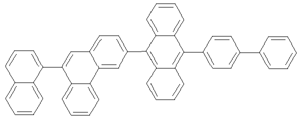 | 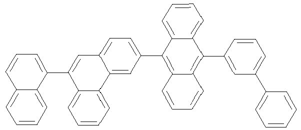 | 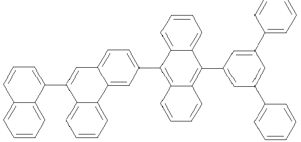 _{^} |
| (19) | (20) | (21) |
| 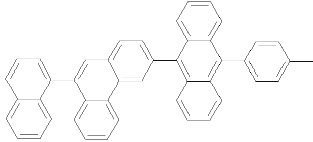 | 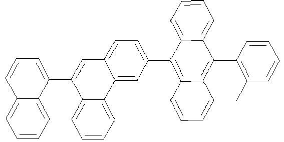 | 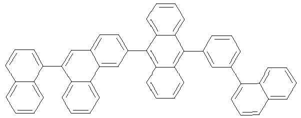 |
| (22)* | (23)* | (24) |
| 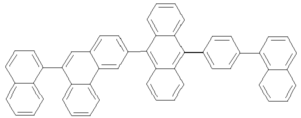 | 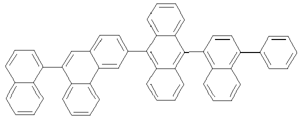 | 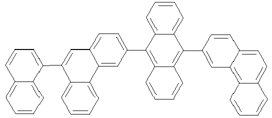 |
| (25) | (26) | (27) |
| 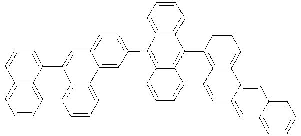 | 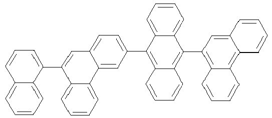 | 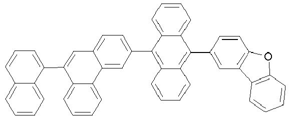 |
| (28) | (29) | (30) |
| 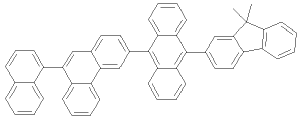 | 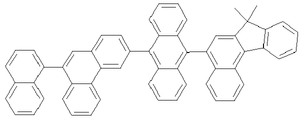 | 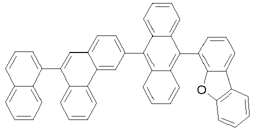 |
| (31) | (32) | (33) |
| 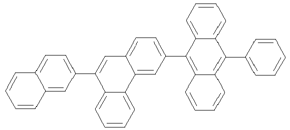 | 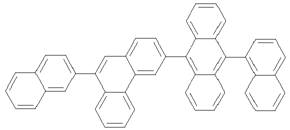 | 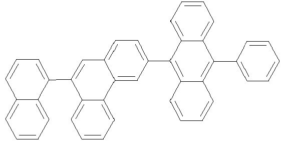 |
| (34)* | (35) | (36)* |
| 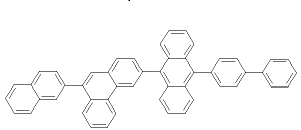 | 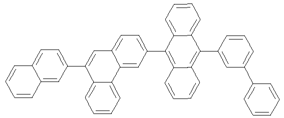 | 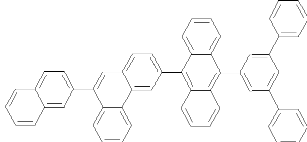 |
| (37) | (38) | (39) |
| 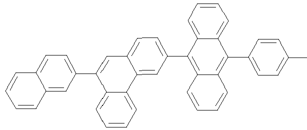 | 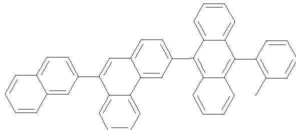 | 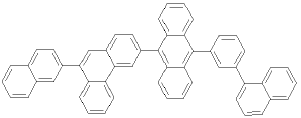 |
| (40)* | (41)* | (42) |
| 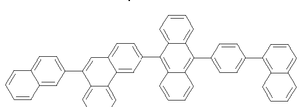 | 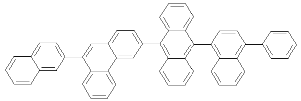 | 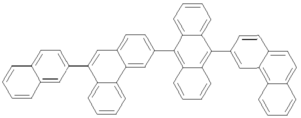 |
| (43) | (44) | (45) |
| 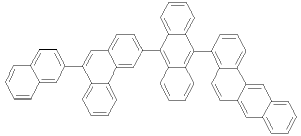 | 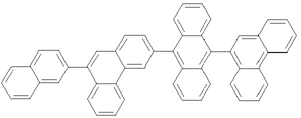 | 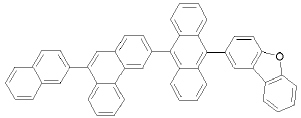 |
| (46) | (47) | (48) |
| 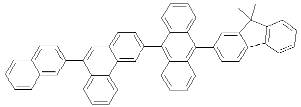 | 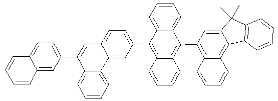 | 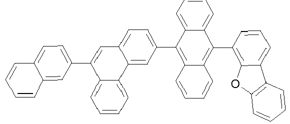 |
| (49) | (50) | (51) |
| 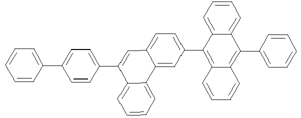 | 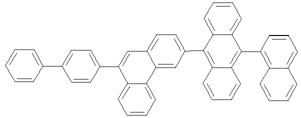 | 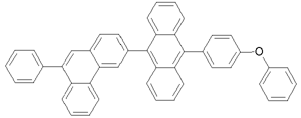 |
| (52)* | (53) | (54) |
| 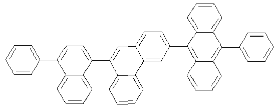 | 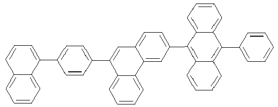 | 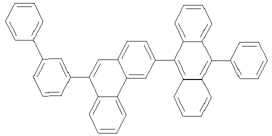 |
| (55)* | (56)* | (57)* |
| 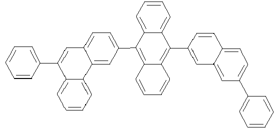 | 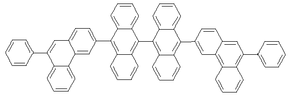 | 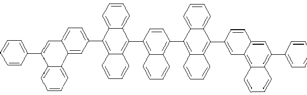 |
| (58) | (59) | (60) |
| 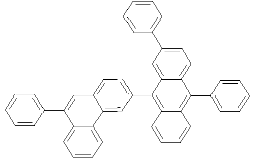 | 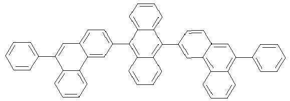 | 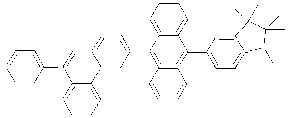 |
| (61)* | (62) | (63)* |
| 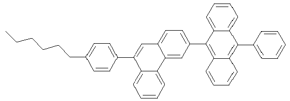 | 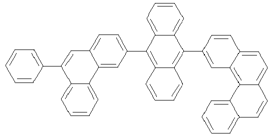 | 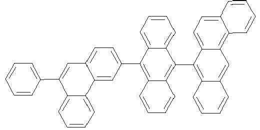 |
| (64)* | (65) | (66) |
| 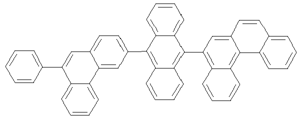 | 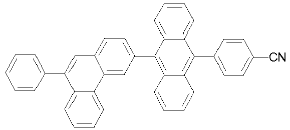 | 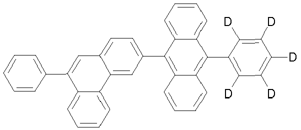 |
| (67) | (68)* | (69)* |
| 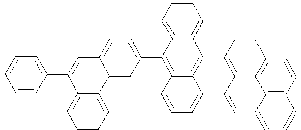 | 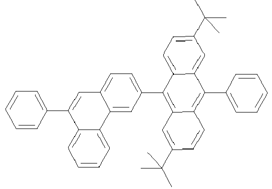 | 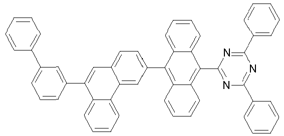 |
| (70) | (71)* | (72) |
| 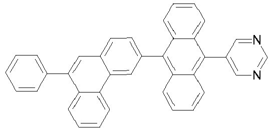 | 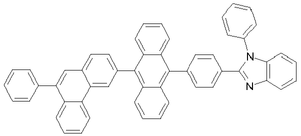 | 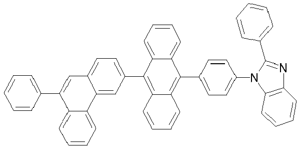 |
| (73) | (74) | (75) |
| 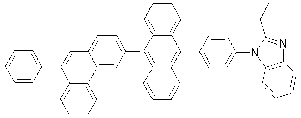 | 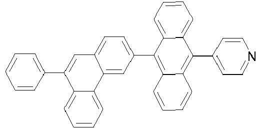 | 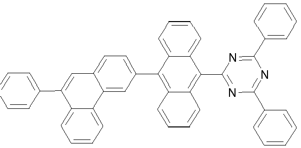 |
| (76) | (77) | (78) |
| 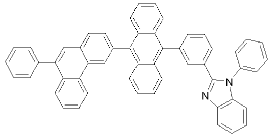 | 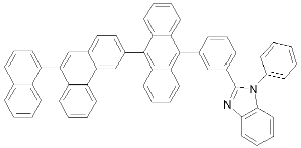 | 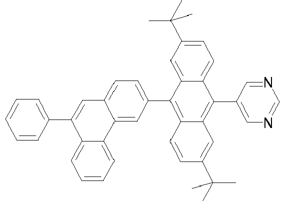 |
| (79) | (80) | (81) |
| 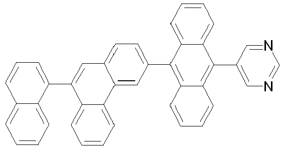 | 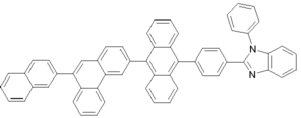 | 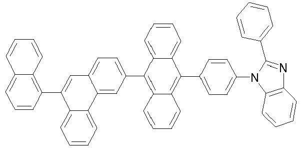 |
| (82) | (83) | (84) |
| 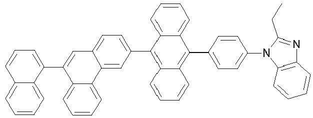 | 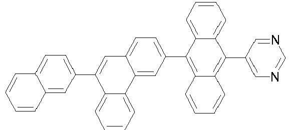 | |
| (85) | (86) | |

| | | |
|---|---|---|
| *Not according to the invention | | |

The compounds can be prepared using known synthesis methods of organic chemistry, such as halogenation, Suzuki coupling and Sonogashira coupling. In the schemes which are shown below, R represents H or any organic radical.

The compounds according to formula (I) are prepared by the two steps of 1) synthesizing the aryl-substituted phenanthrene moiety (Scheme 1), and 2) coupling of this aryl-substituted phenanthrene moiety to an anthracene derivative (Scheme 2). The anthracene derivative is preferably an anthracene which is substituted in 9-position by a reactive group, preferably a halogen group. In a further step, the coupled phenanthrenyl-anthracene may be further substituted in 10-position of the anthracene by a halogenation reaction followed by a Suzuki coupling (route shown in the lower part of Scheme 2).

The preferred detailed synthesis sequence of above step 1) is as follows: An aryl-alkyne derivative is coupled to a tris-halogen substituted phenyl group in a Sonogashira coupling. The resulting alkinyl-phenyl intermediate is then reacted with a phenyl group at one of the remaining halogen-substituted positions on the phenyl moiety of the alkinyl-phenyl intermediate in a Suzuki reaction. The resulting compound then undergoes a carbocyclization reaction, preferably by heating and induced by metal ions, more preferably by heating and in the presence of an iron(III)triflate compound. The obtained aryl-substituted phenanthrene is then further transformed into the respective boronic ester derivative.

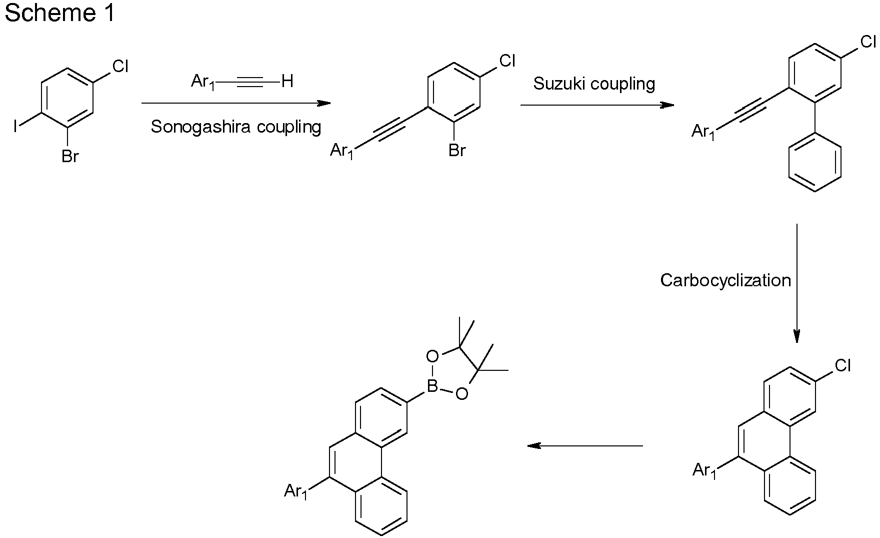

The preferred detailed synthesis sequence of above step 2) is as follows: The phenanthrene boronic ester which was obtained in step 1) is reacted in a Suzuki coupling with an anthracene derivative. In one preferred alternative of this step, the Suzuki coupling is performed with a 9-halogen substituted anthracene which is unsubstituted in the 10-position (route of lower part of Scheme 2). In this case, the coupling product is subsequently brominated and then undergoes a Suzuki coupling in the 10-position of the anthracene moiety. In a second preferred alternative of this step, the Suzuki coupling is performed with a 9-halogen substituted anthracene which is already substituted in the 10-position (route of upper part of Scheme 2). In this case, a compound according to formula (I) is directly obtained. The 9-halogen substituted anthracene which is substituted in the 10-position, which is necessary for this reaction, can be obtained as shown in Scheme 3 below.

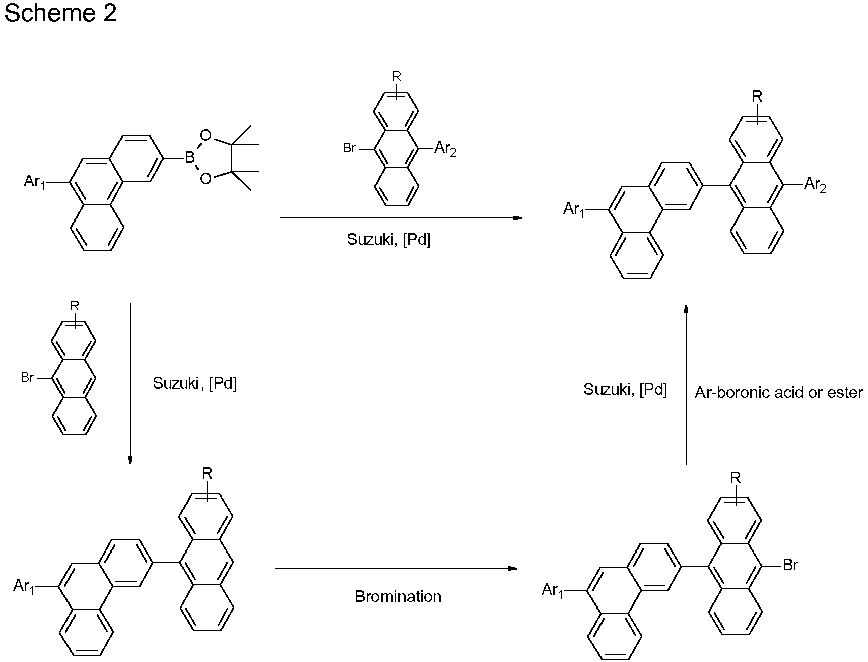

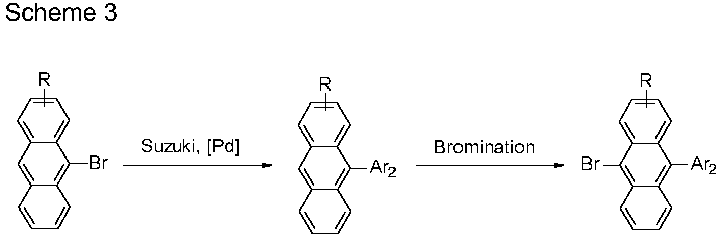

A process for preparation of a compound according to formula (I) is therefore another embodiment of the present invention.

Such process of preparation is characterized in that
- an aryl-substituted phenanthrene derivative is prepared by a sequence comprising a transition metal catalysed coupling reaction and a carbocyclization reaction, and
- in that this aryl-substituted phenanthrene derivative is coupled to an anthracene derivative in a transition metal catalysed coupling reaction.

Preferred embodiments of this method are the ones which are described above.

The compounds according to the invention described above, in particular compounds which are substituted by reactive leaving groups, such as bromine, iodine, chlorine, boronic acid or boronic acid ester, can be used as monomers for the production of corresponding oligomers, dendrimers or polymers. Suitable reactive leaving groups are, for example, bromine, iodine, chlorine, boronic acids, boronic acid esters, amines, alkenyl or alkynyl groups having a terminal C-C double bond or C-C triple bond, oxiranes, oxetanes, groups which undergo a cycloaddition, for example a 1,3-dipolar cycloaddition, such as, for example, dienes or azides, carboxylic acid derivatives, alcohols and silanes.

The invention therefore furthermore relates to oligomers, polymers or dendrimers containing one or more compounds of the formula (I), where the bond(s) to the polymer, oligomer or dendrimer may be localised at any desired positions in formula (I) that are substituted by R¹, R², R³ or R⁴. Depending on the linking of the compound of the formula (I), the compound is a constituent of a side chain of the oligomer or polymer or a constituent of the main chain. An oligomer in the sense of this invention is taken to mean a compound which is built up from at least three monomer units. A polymer in the sense of the invention is taken to mean a compound which is built up from at least ten monomer units. The polymers, oligomers or dendrimers according to the invention may be conjugated, partially conjugated or non-conjugated. The oligomers or polymers according to the invention may be linear, branched or dendritic. In the structures linked in a linear manner, the units of the formula (I) may be linked directly to one another or they may be linked to one another via a divalent group, for example via a substituted or unsubstituted alkylene group, via a heteroatom or via a divalent aromatic or heteroaromatic group. In branched and dendritic structures, for example, three or more units of the formula (I) may be linked via a trivalent or polyvalent group, for example via a trivalent or polyvalent aromatic or heteroaromatic group, to form a branched or dendritic oligomer or polymer.

The same preferences as described above for compounds of the formula (I) apply to the recurring units of the formula (I) in oligomers, dendrimers and polymers.

For the preparation of the oligomers or polymers, the monomers according to the invention are homopolymerised or copolymerised with further monomers. Suitable and preferred comonomers are selected from fluorenes (for example in accordance with EP 842208 or WO 00/22026), spirobifluorenes (for example in accordance with EP 707020, EP 894107 or WO 06/ 061181), para-phenylenes (for example in accordance with WO 1992/ 18552), carbazoles (for example in accordance with WO 04/070772 or WO 2004/113468), thiophenes (for example in accordance with EP 1028136), dihydrophenanthrenes (for example in accordance with WO 2005/014689 or WO 2007/006383), cis- and trans-indenofluorenes (for example in accordance with WO 2004/041901 or WO 2004/113412), ketones (for example in accordance with WO 2005/040302), phenanthrenes (for example in accordance with WO 2005/104264 or WO 2007/017066) or also a plurality of these units. The polymers, oligomers and dendrimers usually also contain further units, for example emitting (fluorescent or phosphorescent) units, such as, for example, vinyltriarylamines (for example in accordance with WO 2007/068325) or phosphorescent metal complexes (for example in accordance with WO 2006/003000), and/or charge-transport units, in particular those based on triarylamines.

The polymers and oligomers according to the invention are generally prepared by polymerisation of one or more types of monomer, at least one monomer of which results in recurring units of the formula (I) in the polymer. Suitable polymerisation reactions are known to the person skilled in the art and are described in the literature. Particularly suitable and preferred polymerisation reactions which result in C-C or C-N links are the following:
(A) SUZUKI polymerisation;
(B) YAMAMOTO polymerisation;
(C) STILLE polymerisation; and
(D) HARTWIG-BUCHWALD polymerisation.

The way in which the polymerisation can be carried out by these methods and the way in which the polymers can then be separated off from the reaction medium and purified is known to the person skilled in the art and is described in detail in the literature, for example in WO 2003/048225, WO 2004/037887 and WO 2004/037887.

For the processing of the compounds according to the invention from the liquid phase, for example by spin coating or by printing processes, formulations of the compounds according to the invention are necessary. These formulations can be, for example, solutions, dispersions or emulsions. It may be preferred to use mixtures of two or more solvents for this purpose. Suitable and preferred solvents are, for example, toluene, anisole, o-, m- or p-xylene, methyl benzoate, mesitylene, tetralin, veratrol, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetol, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The invention therefore furthermore relates to a formulation, in particular a solution, dispersion or emulsion, comprising at least one compound of the formula (I) or at least one polymer, oligomer or dendrimer containing at least one unit of the formula (I), and at least one solvent, preferably an organic solvent. The way in which solutions of this type can be prepared is known to the person skilled in the art and is described, for example, in WO 2002/072714, WO 2003/019694 and the literature cited therein.

The compounds according to the invention are suitable for use in electronic devices, in particular in organic electroluminescent devices (OLEDs). Depending on the substitution, the compounds are employed in different functions and layers.

The compounds according to the invention can be employed in any function in the organic electroluminescent device, for example as matrix material, as emitting material, as hole-transporting material or as electron-transporting material. Preference is given to the use as matrix material in an emitting layer, preferably a fluorescent emitting layer, and the use as electron transporting material in an electron transporting layer or an emitting layer of an organic electroluminescent device.

The invention therefore furthermore relates to the use of a compound of the formula (I) in an electronic device. The electronic device here is preferably selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and particularly preferably organic electroluminescent devices (OLEDs).

The invention furthermore relates to an electronic device comprising at least one compound of the formula (I). The electronic device here is preferably selected from the devices indicated above. Particular preference is given to an organic electroluminescent device comprising anode, cathode and at least one emitting layer, characterised in that at least one organic layer comprises at least one organic compound of the formula (I). Very particular preference is given to an organic electroluminescent device comprising anode, cathode and at least one layer selected from emitting layers and electron transporting layers, comprising at least one compound of the formula (I).

Apart from cathode, anode and the emitting layer, the organic electroluminescent device may also comprise further layers. These are selected, for example, from in each case one or more hole-injection layers, hole-transport layers, hole-blocking layers, electron-transport layers, electron-injection layers, electron-blocking layers, exciton-blocking layers, interlayers, charge-generation layers (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer*)* and/or organic or inorganic p/n junctions.

The sequence of the layers of the organic electroluminescent device is preferably the following:
anode-hole-injection layer-hole-transport layer-emitting layer-electron-transport layer-electron-injection layer-cathode. It is not necessary for all of the said layers to be resent here, and in addition further layers may be present, for example an electron-blocking layer adjacent to the emitting layer on the anode side, or a hole-blocking layer adjacent to the emitting layer on the cathode side.

The organic electroluminescent device according to the invention may comprise a plurality of emitting layers. These emission layers in this case particularly preferably have in total a plurality of emission maxima between 380 nm and 750 nm, resulting overall in white emission, i.e. various emitting compounds which are able to fluoresce or phosphoresce and which emit blue or yellow or green or orange or red light are used in the emitting layers. Particular preference is given to three-layer systems, i.e. systems having three emitting layers, where at least one of these layers preferably comprises at least one compound of the formula (I) and where the three layers exhibit blue, green and orange or red emission (for the basic structure see, for example, WO 2005/011013). It should be noted that, for the generation of white light, an emitter compound used individually which emits in a broad wavelength range may also be suitable instead of a plurality of emitter compounds emitting in colour. The compounds according to the invention may alternatively and/or additionally also be present in an electron transporting layer or in another layer in an organic electroluminescent device of this type.

The compound according to the invention is particularly suitable for use as matrix compound for an emitter compound, preferably a blue-emitting emitter compound, particularly preferably a blue-emitting fluorescent emitter compound. In this case, it is preferred that the compound comprises no heteroaromatic groups. Particularly preferably, in this case, the group Ar² in the compound is selected from phenyl, 1-naphthyl, and aromatic ring systems having 13 to 30 aromatic ring atoms, each of which may be optionally substituted. Even more preferably, group Ar² in this case is optionally substituted phenyl.

The compound according to the invention can also be used as matrix compound for emitter compounds which exhibit thermally activated delayed fluorescence (TADF). The basic principles of the emission mechanism in TADF are disclosed in H. Uoyama et al., Nature 2012, 492, 234.

If the compound according to the invention is employed as matrix material, it can be employed combined with any desired emitting compounds known to the person skilled in the art. It is preferably employed in combination with the preferred emitting compounds indicated below, particularly the preferred fluorescent compounds indicated below.

In the case where the emitting layer of the organic electroluminescent device comprises a mixture of an emitting compound and a matrix compound, the following applies:
The proportion of the emitting compound in the mixture of the emitting layer is preferably between 0.1 and 50.0%, particularly preferably between 0.5 and 20.0%, and very particularly preferably between 1.0 and 10.0%. Correspondingly, the proportion of the matrix material or matrix materials is preferably between 50.0 and 99.9%, particularly preferably between 80.0 and 99.5%, and very particularly preferably between 90.0 and 99.0%.

The indications of the proportions in % in the context of the present application are taken to mean % by vol. if the compounds are applied from the gas phase, and they are taken to mean % by weight if the compounds are applied from solution.

The compound according to the invention can furthermore also be employed as electron-transporting compound in a layer with electron-transporting functionality, such as an electron-transport layer, a hole-blocking layer, an electron-injection layer or an emitting layer. For this purpose, it is preferred for the compound according to the invention to contain one or more substituents selected from electron-deficient heteroaryl groups, such as, for example, triazine, pyrimidine, pyridine, imidazole or benzimidazole. Preferably, in this case, the group Ar² in the compound of formula (I) represents or contains such electron-deficient heteroaryl group.

Generally preferred classes of material for use as corresponding functional materials in the organic electroluminescent devices according to the invention are indicated below.

Suitable phosphorescent emitting compounds are, in particular, compounds which emit light, preferably in the visible region, on suitable excitation and in addition contain at least one atom having an atomic number greater than 20, preferably greater than 38 and less than 84, particularly preferably greater than 56 and less than 80. The phosphorescent emitting compounds used are preferably compounds which contain copper, molybdenum, tungsten, rhenium, ruthenium, osmium, rhodium, iridium, palladium, platinum, silver, gold or europium, in particular compounds which contain iridium, platinum or copper.

For the purposes of the present invention, all luminescent iridium, platinum or copper complexes are regarded as phosphorescent compounds.

Examples of the phosphorescent emitting compounds described above are revealed by the applications WO 2000/70655, WO 2001/41512, WO 2002/ 02714, WO 2002/15645, EP 1191613, EP 1191612, EP 1191614, WO 2005/033244, WO 2005/019373 and US 2005/0258742. In general, all phosphorescent complexes as used in accordance with the prior art for phosphorescent OLEDs and as are known to the person skilled in the art in the area of organic electroluminescent devices are suitable for use in the devices according to the invention. The person skilled in the art will also be able, without inventive step, to employ further phosphorescent complexes in combination with the compounds according to the invention in OLEDs.

Preferred fluorescent emitters, besides the compounds according to the invention, are selected from the class of the arylamines. An arylamine in the sense of this invention is taken to mean a compound which contains three substituted or unsubstituted aromatic or heteroaromatic ring systems bonded directly to the nitrogen. At least one of these aromatic or heteroaromatic ring systems is preferably a condensed ring system, particularly preferably having at least 14 aromatic ring atoms. Preferred examples thereof are aromatic anthracenamines, aromatic anthracenediamines, aromatic pyrenamines, aromatic pyrenediamines, aromatic chrysenamines or aromatic chrysenediamines. An aromatic anthracenamine is taken to mean a compound in which one diarylamino group is bonded directly to an anthracene group, preferably in the 9-position. An aromatic anthracene-diamine is taken to mean a compound in which two diarylamino groups are bonded directly to an anthracene group, preferably in the 9,10-position. Aromatic pyrenamines, pyrenediamines, chrysenamines and chrysenediamines are defined analogously thereto, where the diarylamino groups are preferably bonded to the pyrene in the 1-position or in the 1,6-position. Further preferred emitters are indenofluorenamines or indenofluorene-diamines, for example in accordance with WO 2006/108497 or WO 2006/ 122630, benzoindenofluorenamines or benzoindenofluorenediamines, for example in accordance with WO 2008/006449, and dibenzoindenofluorenamines or dibenzoindenofluorenediamines, for example in accordance with WO 2007/140847, and the indenofluorene derivatives containing condensed aryl groups which are disclosed in WO 2010/012328. Preference is likewise given to the pyrenarylamines disclosed in WO 2012/048780 and WO 2013/185871. Preference is likewise given to the benzoindenofluorenamines disclosed in WO 2014/037077, the benzofluorene-amines disclosed in WO 2014/106522 and the extended indenofluorenes disclosed in WO 2014/111269.

Preferred fluorescent emitting compounds are depicted in the following table:

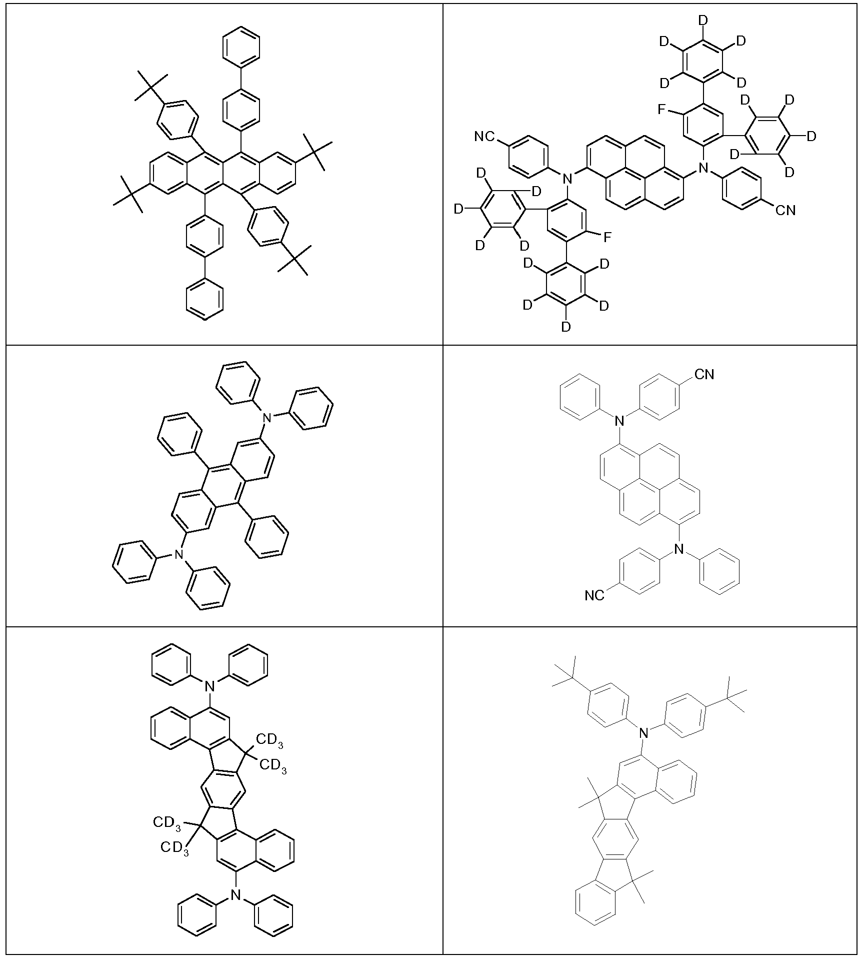

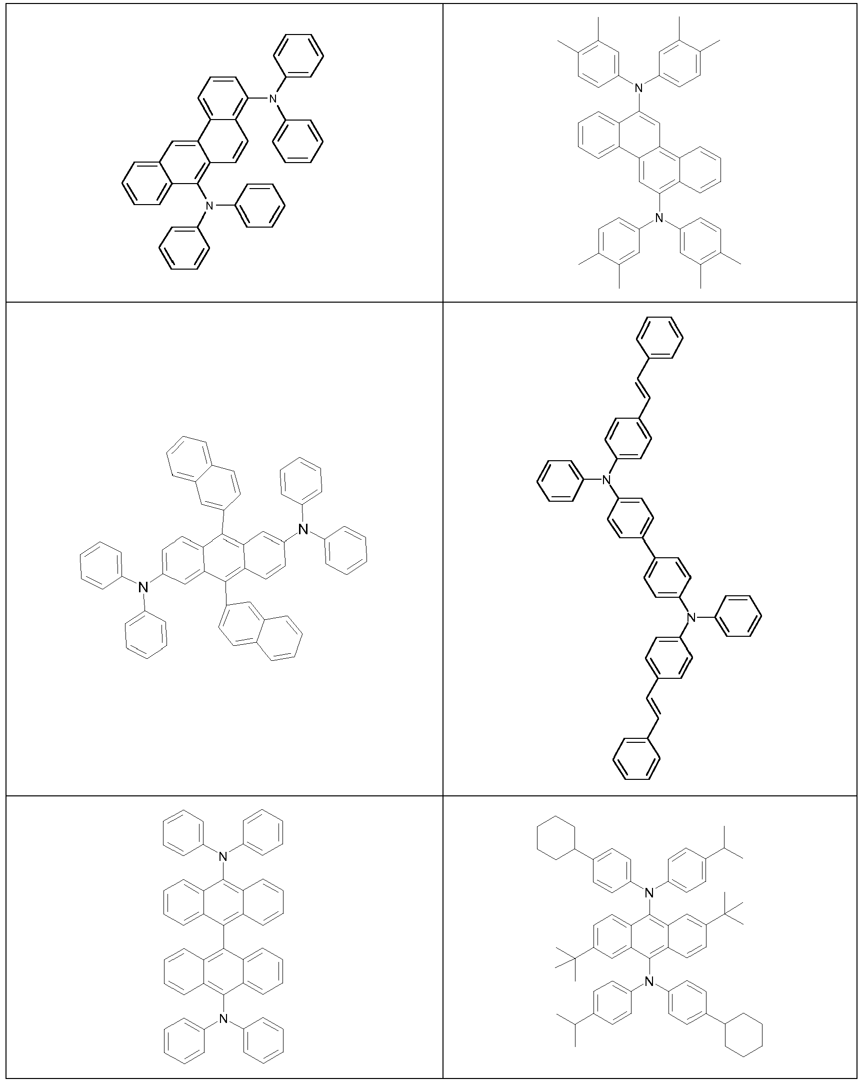

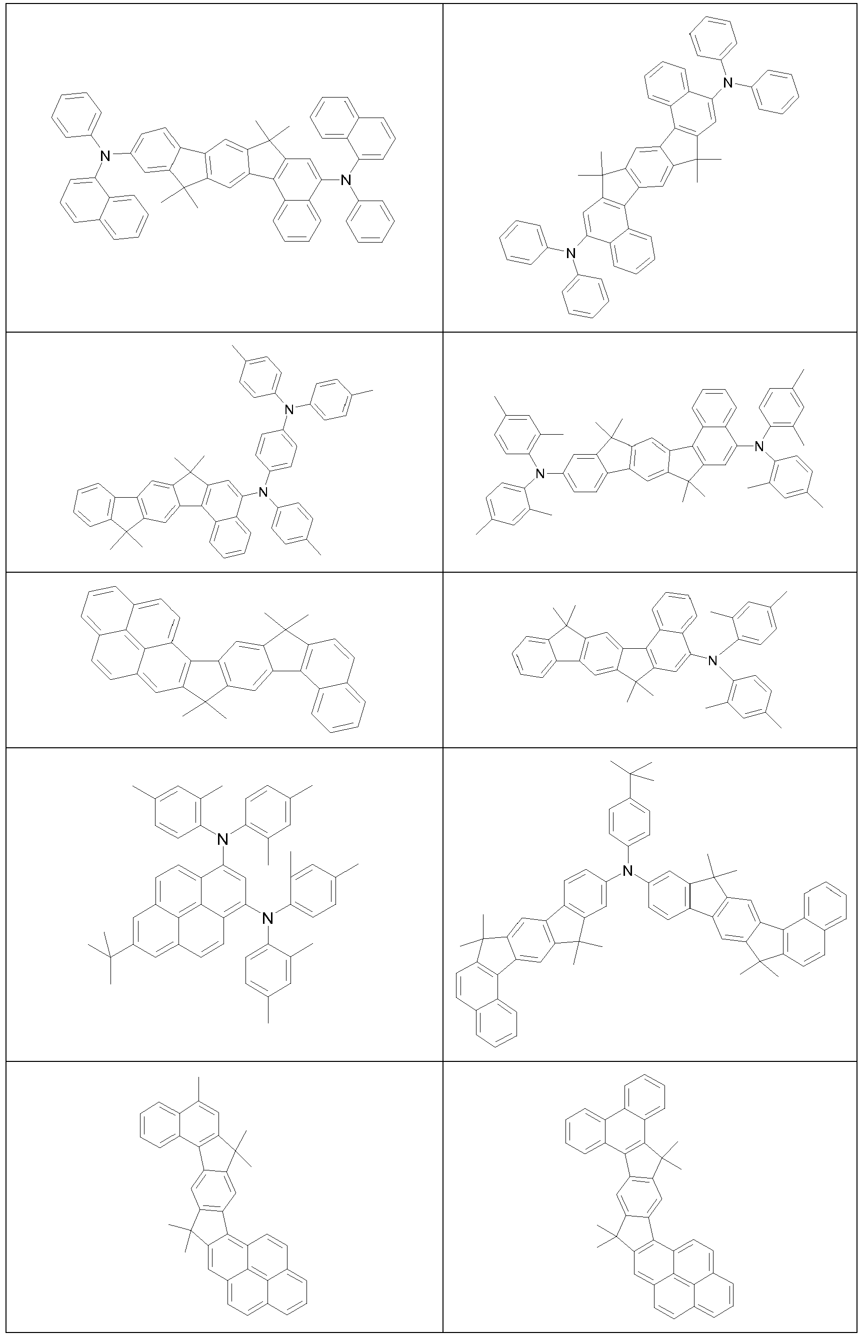

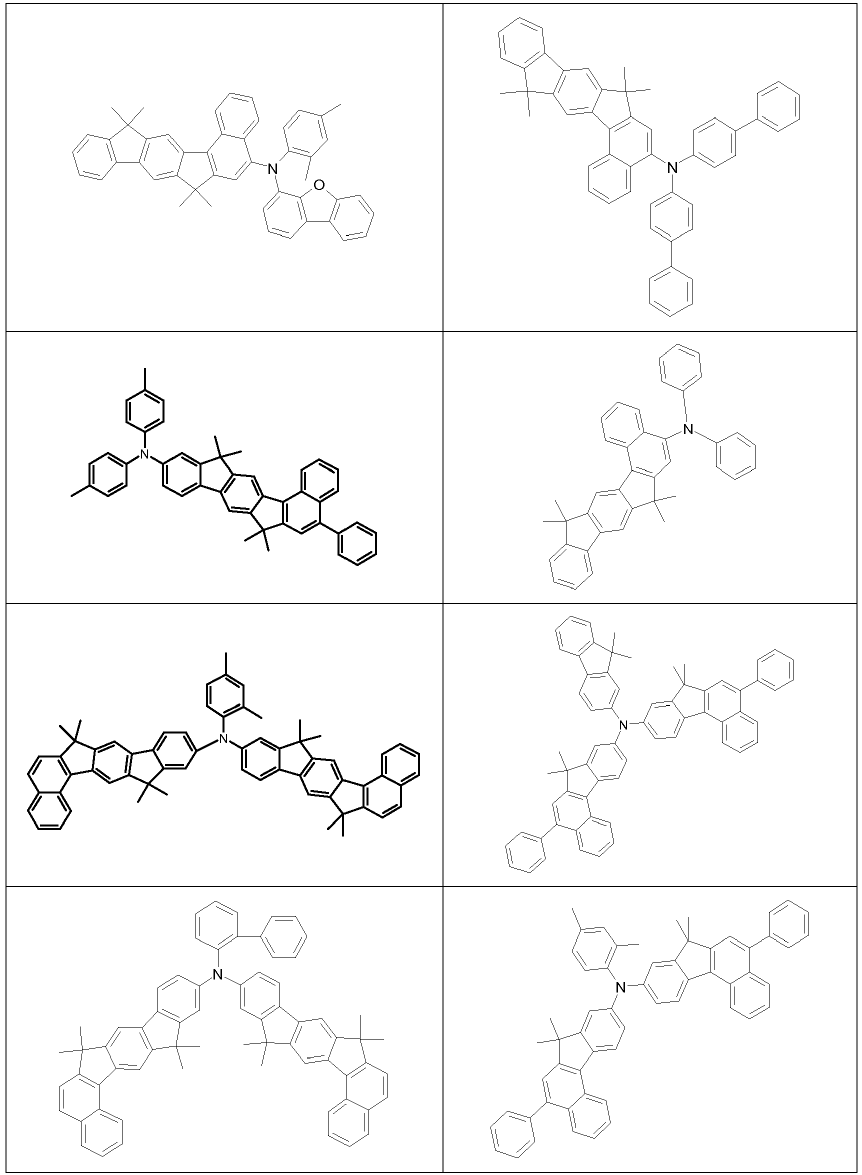

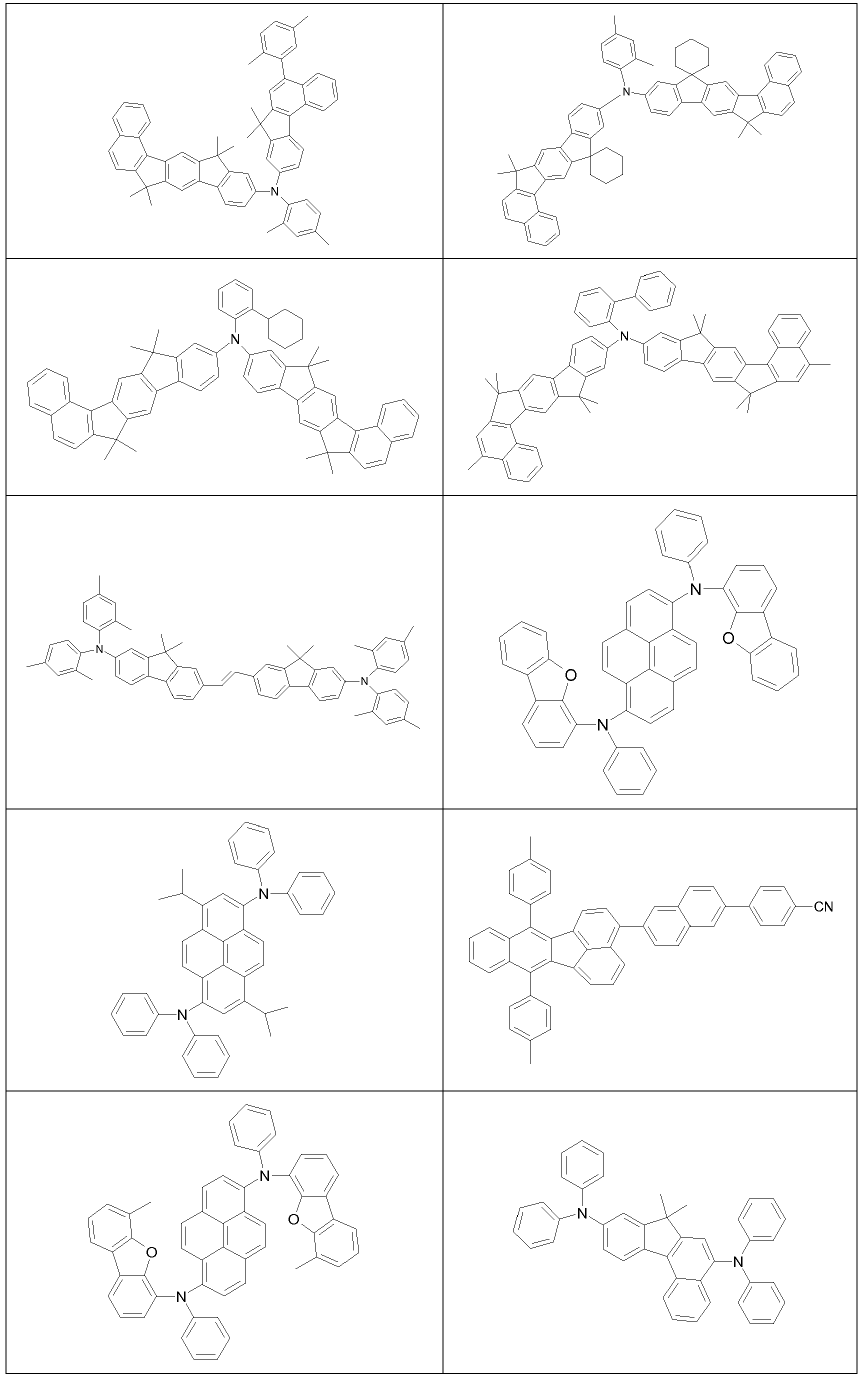

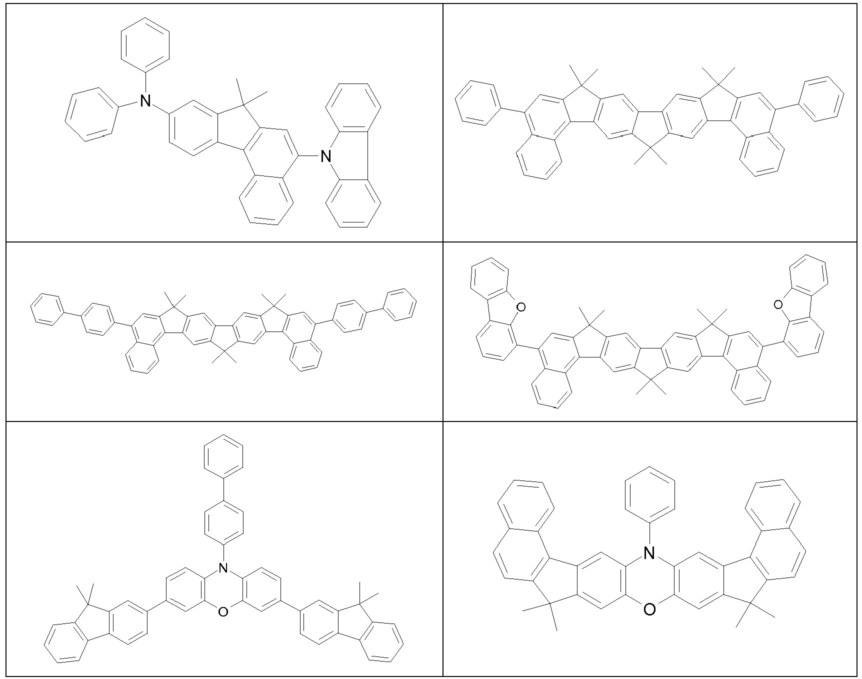

Preferred matrix materials for phosphorescent emitting compounds are aromatic amines, in particular triarylamines, for example in accordance with US 2005/0069729, carbazole derivatives (for example CBP, N,N-bis-carbazolylbiphenyl) or compounds in accordance with WO 2005/039246, US 2005/0069729, JP 2004/288381, EP 1205527 or WO 2008/086851, bridged carbazole derivatives, for example in accordance with WO 2011/ 088877 and WO 2011/128017, indenocarbazole derivatives, for example in accordance with WO 2010/ 136109 and WO 2011/000455, azacarbazole derivatives, for example in accordance with EP 1617710, EP 1617711, EP 1731584, JP 2005/347160, indolocarbazole derivatives, for example in accordance with WO 2007/ 063754 or WO 2008/056746, ketones, for example in accordance with WO 2004/093207 or WO 2010/006680, phosphine oxides, sulfoxides and sulfones, for example in accordance with WO 2005/003253, oligophenylenes, bipolar matrix materials, for example in accordance with WO 2007/137725, silanes, for example in accordance with WO 2005/ 111172, azaboroles or boronic esters, for example in accordance with WO 2006/117052, triazine derivatives, for example in accordance with WO 2010/015306, WO 2007/063754 or WO 2008/056746, zinc complexes, for example in accordance with EP 652273 or WO 2009/ 062578, aluminium complexes, for example BAlq, diazasilole and tetraazasilole derivatives, for example in accordance with WO 2010/054729, and diazaphosphole derivatives, for example in accordance with WO 2010/ 054730.

Preferred matrix materials for use in combination with fluorescent emitting compounds, besides the compounds of the formula (I), are selected from the classes of the oligoarylenes (for example 2,2',7,7'-tetraphenyl-spirobifluorene in accordance with EP 676461 or dinaphthylanthracene), in particular the oligoarylenes containing condensed aromatic groups, the oligoarylenevinylenes (for example DPVBi or spiro-DPVBi in accordance with EP 676461), the polypodal metal complexes (for example in accordance with WO 2004/081017), the hole-conducting compounds (for example in accordance with WO 2004/058911), the electron-conducting compounds, in particular ketones, phosphine oxides, sulfoxides, etc. (for example in accordance with WO 2005/084081 and WO 2005/084082), the atropisomers (for example in accordance with WO 2006/048268), the boronic acid derivatives (for example in accordance with WO 2006/117052) or the benzanthracenes (for example in accordance with WO 2008/ 145239). Particularly preferred matrix materials are selected from the classes of the oligoarylenes, comprising naphthalene, anthracene, benzanthracene and/or pyrene or atropisomers of these compounds, the oligoarylenevinylenes, the ketones, the phosphine oxides and the sulfoxides. Very particularly preferred matrix materials are selected from the classes of the oligoarylenes, comprising anthracene, benzanthracene, benzophenanthrene and/or pyrene or atropisomers of these compounds. An oligoarylene in the sense of this invention is intended to be taken to mean a compound in which at least three aryl or arylene groups are bonded to one another.

Suitable charge-transport materials, as can be used in the hole-injection or hole-transport layer or electron-blocking layer or in the electron-transport layer of the organic electroluminescent device according to the invention, are, for example, the compounds disclosed in Y. Shirota et al., Chem. Rev. 2007, 107(4), 953-1010, or other materials as are employed in these layers in accordance with the prior art.

Examples of preferred hole-transport materials which can be used in a hole-transport, hole-injection or electron-blocking layer in the electroluminescent device according to the invention, besides the compounds of the formula (I), are indenofluorenamine derivatives (for example in accordance with WO 06/122630 or WO 06/100896), the amine derivatives disclosed in EP 1661888, hexaazatriphenylene derivatives (for example in accordance with WO 01/049806), amine derivatives containing condensed aromatic rings (for example in accordance with US 5,061,569), the amine derivatives disclosed in WO 95/09147, monobenzoindenofluorenamines (for example in accordance with WO 08/006449), dibenzoindenofluorenamines (for example in accordance with WO 07/140847), spirobifluorenamines (for example in accordance with WO 2012/034627 or WO 2013/120577), fluorenamines (for example in accordance with WO 2014/015937, WO 2014/015938 and WO 2014/015935), spirodibenzopyranamines (for example in accordance with WO 2013/083216) and dihydroacridine derivatives (for example in accordance with WO 2012/150001).

The cathode of the organic electroluminescent device preferably comprises metals having a low work function, metal alloys or multilayered structures comprising various metals, such as, for example, alkaline-earth metals, alkali metals, main-group metals or lanthanoids (for example Ca, Ba, Mg, Al, In, Mg, Yb, Sm, etc.). Also suitable are alloys comprising an alkali metal or alkaline-earth metal and silver, for example an alloy comprising magnesium and silver. In the case of multilayered structures, further metals which have a relatively high work function, such as, for example, Ag or Al, can also be used in addition to the said metals, in which case combinations of the metals, such as, for example, Ca/Ag, Mg/Ag or Ag/Ag, are generally used. It may also be preferred to introduce a thin interlayer of a material having a high dielectric constant between a metallic cathode and the organic semiconductor. Suitable for this purpose are, for example, alkali metal fluorides or alkaline-earth metal fluorides, but also the corresponding oxides or carbonates (for example LiF, Li₂O, BaF₂, MgO, NaF, CsF, Cs₂CO₃, etc.). Furthermore, lithium quinolinate (LiQ) can be used for this purpose. The layer thickness of this layer is preferably between 0.5 and 5 nm.

The anode preferably comprises materials having a high work function. The anode preferably has a work function of greater than 4.5 eV vs. vacuum. Suitable for this purpose are on the one hand metals having a high redox potential, such as, for example, Ag, Pt or Au. On the other hand, metal/metal oxide electrodes (for example Al/Ni/NiOₓ, Al/PtOₓ) may also be preferred. For some applications, at least one of the electrodes must be transparent or partially transparent in order to facilitate either irradiation of the organic material (organic solar cells) or the coupling-out of light (OLEDs, O-lasers). Preferred anode materials here are conductive mixed metal oxides. Particular preference is given to indium tin oxide (ITO) or indium zinc oxide (IZO). Preference is furthermore given to conductive, doped organic materials, in particular conductive, doped polymers.

The device is appropriately (depending on the application) structured, provided with contacts and finally sealed, since the lifetime of the devices according to the invention is shortened in the presence of water and/or air.

In a preferred embodiment, the organic electroluminescent device according to the invention is characterised in that one or more layers are applied by means of a sublimation process, in which the materials are applied by vapour deposition in vacuum sublimation units at an initial pressure of less than 10⁻⁵ mbar, preferably less than 10⁻⁶ mbar. However, it is also possible here for the initial pressure to be even lower, for example less than 10⁻⁷ mbar.

Preference is likewise given to an organic electroluminescent device, characterised in that one or more layers are applied by means of the OVPD (organic vapour phase deposition) process or with the aid of carrier-gas sublimation, in which the materials are applied at a pressure of between 10⁻⁵ mbar and 1 bar. A special case of this process is the OVJP (organic vapour jet printing) process, in which the materials are applied directly through a nozzle and are thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, screen printing, flexographic printing, nozzle printing or offset printing, but particularly preferably LITI (light induced thermal imaging, thermal transfer printing) or ink-jet printing.

For the production of an organic electroluminescent device according to the invention, it is furthermore preferred to apply one or more layers from solution and one or more layers by a sublimation process.

In accordance with the invention, the electronic devices comprising one or more compounds according to the invention can be employed in displays, as light sources in lighting applications and as light sources in medical and/or cosmetic applications (for example light therapy).

### Working examples

### A) Synthesis of compounds

The following syntheses are generally performed under a protective gas atmosphere and with dried solvents. 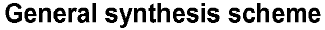 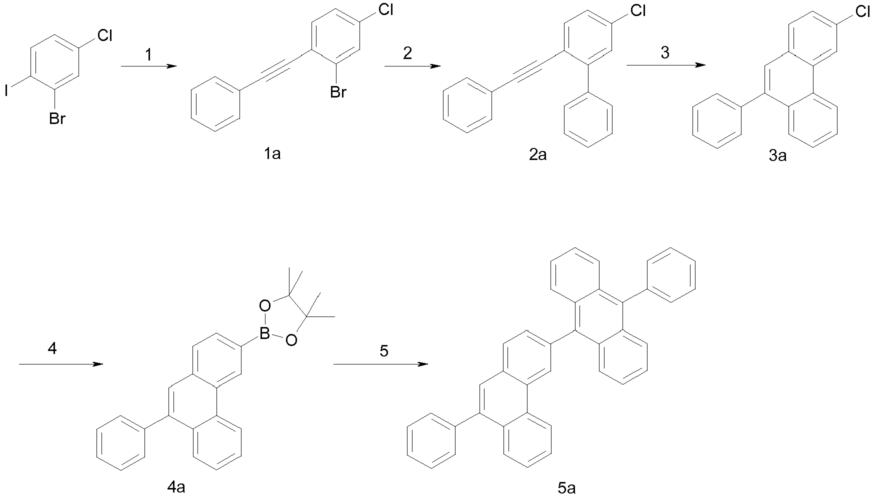
Reaction 1: Sonogashira coupling, Copper(I) catalysis, Pd catalysis
Reaction 2: Suzuki reaction, Pd catalysis
Reaction 3: Metal-induced carbocyclization, Iron(III) triflate
Reaction 4: Borane, Pd catalysis
Reaction 5: Br-anthracene derivative, Pd catalysis, Suzuki reaction

### Example 1a:

50 g (0.16 mol) 2-bromo-4-chloro-1-iodobenzene, 17.3 mL (0.16 mol) phenylacetylene, 1.66 g (2.4 mmol) bis(triphenylphosphine)palladium(II) chloride and 0.3 g (1.6 mmol) copper(I) iodide are mixed in 500 mL of triethylamine and stirred for 2 h at 80°C. Then, the mixture is left to cool to room temperature and 200 mL of saturated aqueous ammonium chloride solution and 400 mL of toluene are added. The phases are separated. The organic phase is washed multiple times with water, and then filtered over silica. After crystallization from ethanol, the product is obtained as a solid. Yield: 38 g (0.13 mol; 83 %).

In analogous manner, the following compounds can be obtained:

| | starting material | compound **1** | yield |
|---|---|---|---|
| 1b | 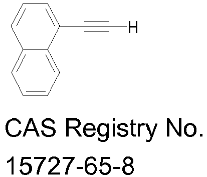 | 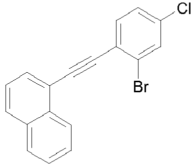 | 72 % |
| 1c | 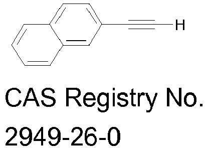 | 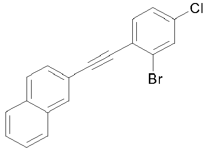 | 81 % |

### Example 2a:

36 g (0.13 mol) **1a,** 16.5 g (0.13 mol) phenylboronic acid, 1.85 g (2.6 mmol) bis(triphenylphosphine)palladium(II) chloride and 27.6 g (0.26 mol) sodium carbonate are mixed in 700 mL toluene/ethanol/water (2:1:1) and are heated under reflux for 5 h. After cooling the mixture to room temperature, 300 mL of water and 300 mL of toluene are added. The phases are separated. The organic phase is washed multiple times with water and the solvent is removed in vacuo. The crude product is purified by column chromatography on silica (heptane). The desired product is obtained as a colorless solid. Yield: 23 g (0.8 mmol, 62 %).

In analogous manner, the following compounds can be obtained:

| | Starting material | compound **2** | yield |
|---|---|---|---|
| 2b | 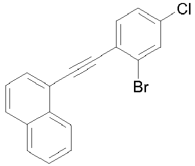 | 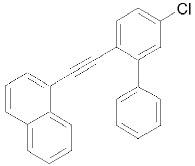 | 85 % |
| 2c | 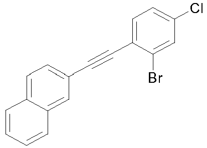 | 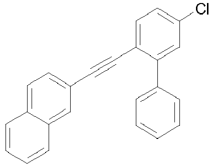 | 78 % |

### Example 3a:

22.2 g (0.077 mol) **2a** and 773 mg (1.5 mmol) iron(III) triflate are stirred for 3 hours at 80°C in 400 mL dichloroethane. Then, the mixture is filtrated over silica, and the solvent is evaporated in vacuo. Yield: 22 g (76 mmol; 99 %).

In analogous manner, the following compounds can be obtained:

| | Starting material | compound **3** | yield |
|---|---|---|---|
| 3b | 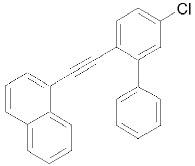 | 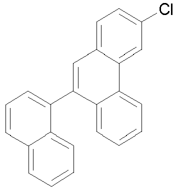 | 97 % |
| 3c | 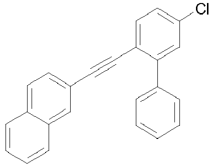 | 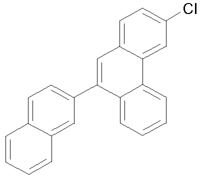 | 96 % |

### Example 4a:

22 g (76 mmol) **3a,** 23.2 g (91 mmol) bis(pinacolato)diboran, 1.12 g (1.5 mmol) bis(tricyclohexylphosphine)palladium(II) chloride and 12.7 g (130 mmol) potassium acetate are stirred for 16 h at 90°C in 300 mL dioxane, and then allowed to cool to room temperature. Afterwards, the mixture is filtrated first over aluminium oxide, and then over silica, and washed with toluene. The solvents are removed in vacuo, and the residue is recrystallized from heptane. The product is isolated as a colorless solid in a yield of 19.6 g (51.5 mmol; 77 %).

In analogous manner, the following compounds can be obtained:

| | Starting material | compound **4** | yield |
|---|---|---|---|
| 4b | 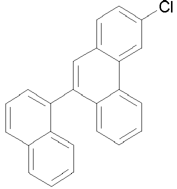 | 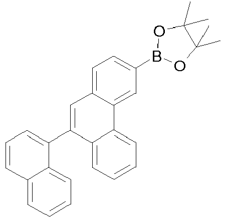 | 65 % |
| 4c | 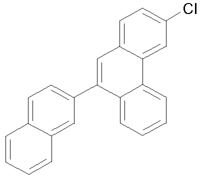 | 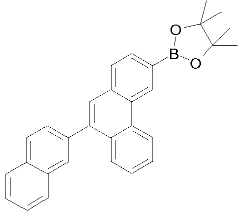 | 83 % |

### Example 5a:

18.9 g (50 mmol) **4a,** 15.5 g (47 mmol) 9-bromo-10-phenylanthracene, 1 g (0.9 mmol) tetrakis(triphenylphosphine)palladium(0) and 17.9 g (0.17 mmol) sodium carbonate are heated under reflux in 600 ml toluene/ethanol/water (2:1:1) for 16 h. After cooling the mixture to room temperature, 200 mL of toluene is added and the phases are separated. The organic phase is washed multiple times with water and the solvent is removed in vacuo. Afterwards, the organic phase is filtrated over aluminium oxide. The product is further purified by recrystallization from toluene and subsequent sublimation. Yield: 7.8 g (15.4 mmol; 33 %).

In analogous manner, the following compounds can be obtained:

| | Starting material (phenanthrene derivative) | Starting material (anthracene derivative) | compound **5** | yield |
|---|---|---|---|---|
| 5b | 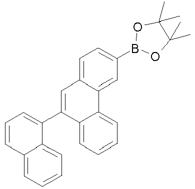 | 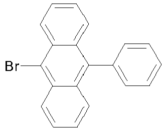 | 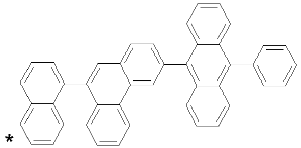 | 52 % |
| | | CAS Registry Num. 23674-20-6 | | |
| 5c | 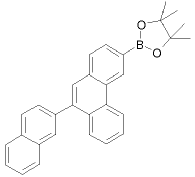 | 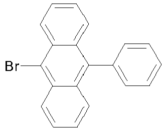 | 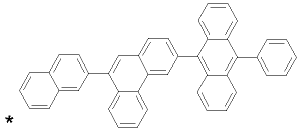 | 41 % |
| | | CAS Registry Num. 23674-20-6 | | |
| 5d | 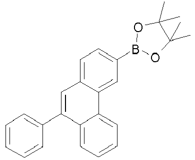 | 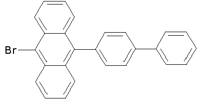 | 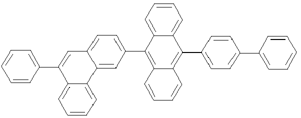 | 40 % |
| | | CAS Registry Num. 400607-05-8 | | |
| 5e | 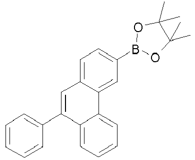 | 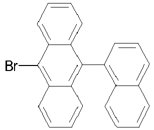 | 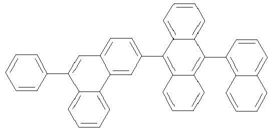 | 49 % |
| | | CAS Registry Num. 400607-04-7 | | |
| 5f | 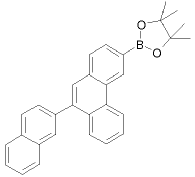 | 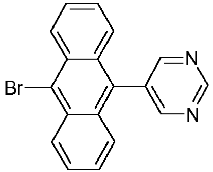 | 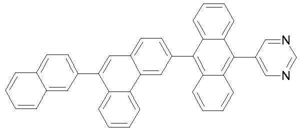 | 38 % |
| | | CAS Registry Num.1304008-79-4 | | |

| | | | | |
|---|---|---|---|---|
| *Not according to the invention | | | | |

### B) Device Examples

### Fabrication of OLED devices

The manufacturing of the OLEDs is performed accordingly to WO 04/05891 with adapted film thicknesses and layer sequences. The following examples V1 to E7 (see Table 1) show data of various OLEDs.

### Substrate pre-treatment of examples V1-E8:

Glass plates with structured ITO (50 nm, indium tin oxide) are coated with 20 nm PEDOT:PSS (Poly(3,4-ethylenedioxythiophene) poly(styrene-sulfonate, CLEVIOS™ P VP AI 4083 from Heraeus Precious Metals GmbH Germany, spin-coated from a water-based solution) and form the substrates on which the OLEDs are processed.

The OLEDs have in principle the following layer structure:
- Substrate,
- ITO (50 nm),
- Buffer (20 nm),
- Hole injection layer (HTL1 95%, HIL 5%) (20 nm),
- Hole transporting layer (HTL2) (20 nm),
- Emissive layer (EML) (20 nm),
- Electron transporting layer (see table 2, EIL 50%) (30 nm),
- Electron injection layer (EIL) (3 nm),
- Cathode.
The cathode is formed by an aluminium layer with a thickness of 100 nm. The materials used for the OLED fabrication are presented in Table 1.

All materials are applied by thermal vapour deposition in a vacuum chamber. The emission layer here always consists of at least one matrix material (host material=H) and an emitting dopant (emitter=D), which is admixed with the matrix material or matrix materials in a certain proportion by volume by co-evaporation. An expression such as H1:D1 (95%:5%) here means that material H1 is present in the layer in a proportion by volume of 95%, whereas D1 is present in the layer in a proportion of 5%. Analogously, the electron-transport layer may also consist of a mixture of two or more materials.

The OLEDs are characterised by standard methods. For this purpose, the electroluminescence spectra, the current efficiency (measured in cd/A) and the external quantum efficiency (EQE, measured in % at 1000 cd/m²) are determined from current/voltage/luminance characteristic lines (IUL characteristic lines) assuming a Lambertian emission profile. The electroluminescence (EL) spectra are recorded at a luminous density of 1000 cd/m² and the CIE 1931 x and y coordinates are then calculated from the EL spectrum. For all experiments, the lifetime LT95 is determined. The lifetime LT95 @ 1000 cd/m² is defined as the time after which the initial luminous density of 1000 cd/m² has dropped by 5%. The device data of various OLEDs is summarized in Table 2. The examples starting with a "V" are comparison examples according to the state-of-the-art. The examples starting with an "E" represent OLEDs according to the invention.

In the following section several examples are described in more detail to show the advantages of the inventive OLEDs.

### Use of inventive compounds as host material in fluorescent OLEDs

The inventive compounds, represented by H1 and H2, are expecially suitable as host materials in fluorescent blue emissive layers of OLEDs. Comparison examples for the state-of-the-art are represented by VH-1 and VH-2, either doped by fluorescent emitters D1 or D2.

The use of the inventive compound as a host material with a single substitution of a phenyl at the phenanthren (H1 in devices E3 and E4) results in significantly improved OLED device date compared to the state-of-the-art, where the phenanthren is substituted two times by a phenyl ring (VH-1 in devices V1 and V2).

Furthermore, the inventive compound with a 1-naphthyl substitution (H2) yields improved EQE and lifetime (devices E7 and E8) compared to the state-of-the-art compound which bears a 2-naphthyl group in the respective position (VH-2 in devices V5 and V6).
In summary, the use of the inventive compounds as host material results in significantly improved OLED device data compared to state-of-the-art materials, especially with respect to external quantum efficiency and device lifetime.

In addition, the inventive compounds are also suitable as electron transporting materials e.g. in fluorescent blue emissive layers of OLEDs, here represented by ETL2. The use of the inventive compound as electron transporting material results in competitive OLED device data, compare device example E9 with state-of-the-art electron transporting material in device V1.

| **Table 1: Chemical structures of the OLED materials** | |
|---|---|
| 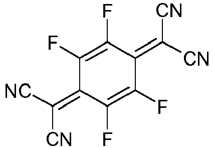 | 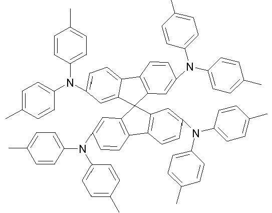 |
| HIL | HTL1 |
| 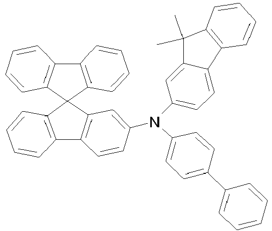 | 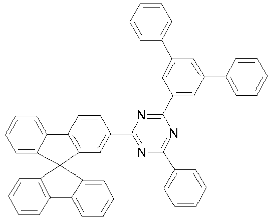 |
| HTL2 | VETL1 |
| 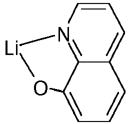 | 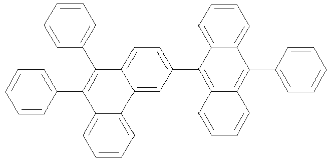 |
| EIL | VH-1** |
| 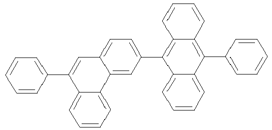 | 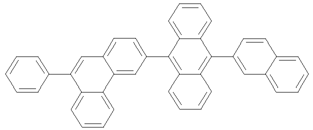 |
| H1* | VH-2** |
| 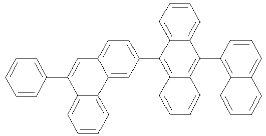 | 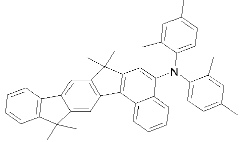 |
| H2 | D1 |
| 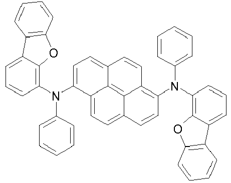 | 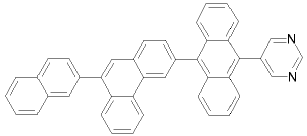 |
| D2 | ETL2 |

| | |
|---|---|
| *Not according to the invention **Compound provided for comparative purposes only | |

| | **Tabelle 2: Device data of OLEDs** | | | | | | |
|---|---|---|---|---|---|---|---|
| ***Example*** | ***Host*** | ***Dopant*** | ***ETL*** | ***EQE* @ *1000 cd*/*m²*** | ***LD95* @ *1000cd*/*m²*** | ***CIE*** | |
| | *95* % | *5 %* | *50%* | % | *[h]* | *x* | *y* |
| V1 | VH-1 | D1 | VETL1 | 5.5 | 40 | 0.14 | 0.14 |
| V2 | VH-1 | D2 | VETL1 | 5.7 | 50 | 0.14 | 0.10 |
| E3 | H-1 | D1 | VETL1 | 7.2 | 75 | 0.15 | 0.13 |
| E4 | H-1 | D2 | VETL1 | 7.5 | 100 | 0.15 | 0.09 |
| V5 | VH-2 | D1 | VETL1 | 6.6 | 70 | 0.14 | 0.14 |
| V6 | VH-2 | D2 | VETL1 | 7.0 | 85 | 0.14 | 0.10 |
| E7 | H-2 | D1 | VETL1 | 6.9 | 90 | 0.14 | 0.14 |
| E8 | H-2 | D2 | VETL1 | 7.3 | 105 | 0.14 | 0.10 |
| E9 | VH-1 | D1 | ETL2 | 5.6 | 35 | 0.14 | 0.14 |

## Claims

1. Compound according to a formula (I) 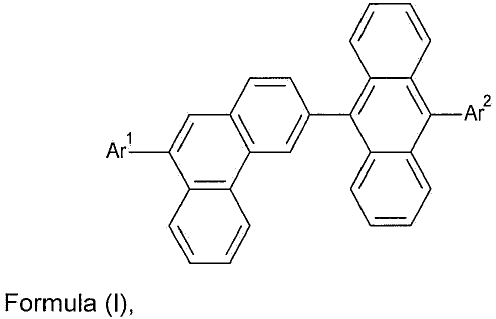 where the phenanthrene group may be substituted by radicals R³ in the free positions, and the anthracene group may be substituted by radicals R⁴ in the free positions, and where the following applies to the variables occuring:
Ar¹ is selected from aryl groups having 6 to 10 aromatic ring atoms, which may be substituted by radicals R¹, or heteroaryl groups having 5 to 18 aromatic ring atoms, which may be substituted by radicals R¹;
Ar² is selected from 1-naphthyl, which may be substituted by radicals R², or aromatic ring systems having 13 to 30 aromatic ring atoms, which may be substituted by radicals R², or heteroaromatic ring systems having 5 to 30 aromatic ring atoms, which may be substituted by radicals R²;
R¹, R², R⁴ is selected, identically or differently at each occurrence, from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, or heteroaromatic ring systems having 5 to 40 aromatic ring atoms; where two or more radicals R¹, R² or R⁴ may be connected to each other to form a ring; where the said alkyl, alkoxy, alkenyl and alkynyl groups and the said aromatic and heteroaromatic ring systems may in each case be substituted by one or more radicals R⁵, and where one or more CH₂ groups in the said alkyl, alkoxy, alkenyl and alkynyl groups may in each case be replaced by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂;
R³ is selected, identically or differently on each occurrence, from H, D, F, CN, straight-chain alkyl or alkoxy groups having 1 to 20 C atoms, branched or cyclic alkyl or alkoxy groups having 3 to 20 C atoms, or alkenyl or alkynyl groups having 2 to 20 C atoms, where two or more radicals R³ may be connected to each other to form a ring; where the said alkyl, alkoxy, alkenyl and alkynyl groups may in each case be substituted by one or more radicals R⁵, and where one or more CH₂ groups in the said alkyl, alkoxy, alkenyl and alkynyl groups may in each case be replaced by -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO or SO₂;
R⁵ is selected, identically or differently at each occurrence, from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, or heteroaromatic ring systems having 5 to 40 aromatic ring atoms; where the said alkyl, alkoxy, alkenyl and alkynyl groups and the said aromatic and heteroaromatic ring systems may in each case be substituted by one or more radicals R⁶, and where one or more CH₂ groups in the said alkyl, alkoxy, alkenyl and alkynyl groups may in each case be replaced by -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO or SO₂;
R⁶ is selected, identically or differently at each occurrence, from H, D, F, CN, alkyl groups having 1 to 20 C atoms, aromatic ring systems having 6 to 40 C atoms, or heteroaromatic ring systems having 5 to 40 aromatic ring atoms, where the said alkyl groups, aromatic ring systems and heteroaromatic ring systems may be substituted by F and CN.

2. Compound according to claim 1, **characterized in that** Ar¹ is a phenyl or a naphthyl group, which may each be substituted by one or more radicals R¹.

3. Compound according to claim 1 or 2, **characterized in that** Ar² is selected from 1-naphthyl, fluorenyl, benzofluorenyl, indenofluorenyl, spirobifluorenyl, benzoindenofluorenyl, phenanthrenyl, anthracenyl, pyrenyl, benzanthracenyl, and benzophenanthrenyl, which may be substituted by one or more radicals R², or heteroaromatic ring systems having 5 to 30 aromatic ring atoms, which may be substituted by radicals R².

4. Compound according to one or more of claims 1 to 3, **characterized in that** Ar² is a heteroaromatic ring system having 5 to 18 aromatic ring atoms, which may be substituted by radicals R².

5. Compound according to one or more of claims 1 to 4, **characterized in that** it conforms to formula (I-2) 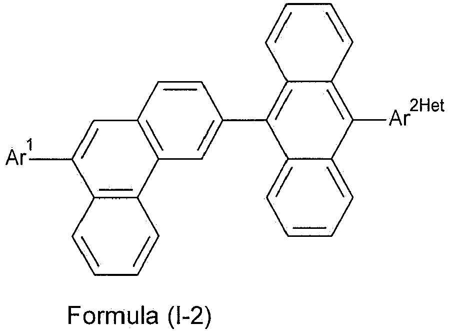 where the phenanthrene group may be substituted by radicals R³ in the free positions, and the anthracene group may be substituted by radicals R⁴ in the free positions, and
where the variables occurring are defined as in claim 1 or 2, and:
Ar^{2Het} is a heteroaromatic ring system having 5 to 18 aromatic ring atoms, which may be substituted by radicals R².

6. Compound according to one or more of claims 1 to 5, **characterized in that** R¹ is selected, identically or differently on each occurrence, from H, D, F, CN, Si(R⁵)₃, straight-chain alkyl or alkoxy groups having 1 to 20 C atoms, or branched or cyclic alkyl or alkoxy groups having 3 to 20 C atoms; where the said alkyl and alkoxy groups may in each case be substituted by one or more radicals R⁵.

7. Compound according to one or more of claims 1 to 6, **characterized in that** radicals R³ are selected, identically or differently on each occurrence, from H, D, F, CN, straight-chain alkyl or alkoxy groups having 1 to 20 C atoms, or branched or cyclic alkyl or alkoxy groups having 3 to 20 C atoms, where the said alkyl or alkoxy groups may in each case be substituted by one or more radicals R⁵.

8. Compound according to one or more of claims 1 to 7, **characterized in that** radicals R³ are H or D.

9. Process for preparation of a compound according to formula (I), **characterized in that**
- an aryl-substituted phenanthrene derivative is prepared by a sequence comprising a transition metal catalysed coupling reaction and a carbocyclization reaction, and
- **in that** this aryl-substituted phenanthrene derivative is coupled to an anthracene derivative in a transition metal catalysed coupling reaction.

10. Oligomer, polymer or dendrimer containing one or more compounds according to one or more of Claims 1 to 8, where the bond(s) to the polymer, oligomer or dendrimer may be localised at any desired positions in formula (I) which are substituted by R¹, R², R³ or R⁴.

11. Formulation comprising at least one compound according to one or more of Claims 1 to 8 or at least one oligomer, polymer or dendrimer according to Claim 10 and at least one solvent.

12. Electronic device selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), comprising at least one compound according to one or more of Claims 1 to 8 or at least one oligomer, polymer or dendrimer according to Claim 10.

13. Electronic device according to Claim 12, selected from organic electroluminescent devices, comprising anode, cathode, emitting layer and optionally further organic layers, **characterised in that** the at least one compound or the at least one oligomer, polymer or dendrimer is present in an emitting layer or in a layer with electron transporting function.

14. Organic electroluminescent device according to Claim 13, **characterised in that** the at least one compound or the at least one oligomer, polymer or dendrimer is present as a matrix material in combination with a fluorescent emitter in an emitting layer, or is present as an electron transporting material in a layer with electron transporting function.

15. Use of a compound according to one or more of Claims 1 to 8 or an oligomer, polymer or dendrimer according to Claim 10 in an electronic device.

## Patentansprüche

1. Verbindung nach einer Formel (I): 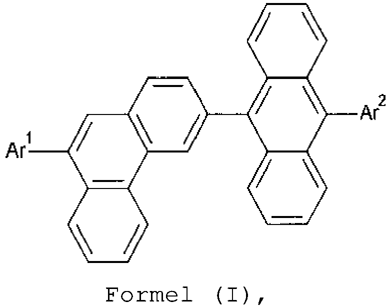 wobei die Phenanthrengruppe in den freien Positionen durch Reste R³ substituiert sein kann, und die Anthracengruppe in den freien Positionen durch Reste R⁴ substituiert sein kann, und wobei für die vorkommenden Variablen folgendes gilt:
Ar¹ ist ausgewählt aus Arylgruppen mit 6 bis 10 aromatischen Ringatomen, die durch Reste R¹ substituiert sein können, oder Heteroarylgruppen mit 5 bis 18 aromatischen Ringatomen, die durch Reste R¹ substituiert sein können;
Ar² ist ausgewählt aus 1-Naphthyl, das durch Reste R² substituiert sein kann, oder aromatischen Ringsystemen mit 13 bis 30 aromatischen Ringatomen, die durch Reste R² substituiert sein können, oder heteroaromatischen Ringsystemen mit 5 bis 30 Kohlenstoffatomen, die durch Reste R² substituiert sein können;
R¹, R², R⁴, die bei jedem Vorkommen identisch oder unterschiedlich sein können, sind ausgewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, oder heteroaromatischen Ringsystemen mit 5 bis 40 aromatischen Ringatomen; wobei zwei oder mehr Reste R¹, R² oder R⁴ miteinander verbunden sein können, um einen Ring zu bilden; wobei die Alkyl-, Alkoxy-, Alkenyl- und Alkinylgruppen und die aromatischen und heteroaromatischen Ringsysteme in jedem Fall durch einen oder mehrere Reste R⁵ substituiert sein können, und wobei eine oder mehrere Gruppen CH₂ in den Alkyl-, Alkoxy-, Alkenyl- und Alkinylgruppen in jedem Fall durch -R⁵C=CR⁵-, -C≡C-, Si(R⁵)₂, C=O, C=NR⁵, -C(=O) O-, -C(=O)NR⁵-, NR⁵, P(=O)(R⁵), -O-, -S-, SO oder SO₂ ersetzt werden kann bzw. können;
R³, das bei jedem Vorkommen identisch oder unterschiedlich sein kann, ist ausgewählt aus H, D, F, CN, geradkettigen Alkyl- oder Alkoxygruppen mit 1 bis 20 C-Atomen, geradkettigen oder cyclischen Alkyl- oder Alkoxygruppen mit 3 bis 20 C-Atomen, oder Alkenyl- oder Alkinylgruppen mit 2 bis 20 C-Atomen, wobei zwei oder mehr Reste R³ miteinander verbunden sein können, um einen Ring zu bilden; wobei die Alkyl-, Alkoxy-, Alkenyl- und Alkinylgruppen in jedem Fall durch einen oder mehrere Reste R⁵ substituiert sein können, und wobei eine oder mehrere Gruppen CH₂ in den Alkyl-, Alkoxy-, Alkenyl- und Alkinylgruppen in jedem Fall durch -R⁵C=CR⁵-,-C≡C-, Si(R⁵)₂, C=O, C=NR⁵, -C(=O)O-, -C(=O)NR⁵-, NR⁵, P(=O)(R⁵), -O-, -S-, SO oder SO₂ ersetzt werden kann bzw. können;
R⁵, das bei jedem Vorkommen identisch oder unterschiedlich sein kann, ist ausgewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, oder heteroaromatischen Ringsystemen mit 5 bis 40 aromatischen Ringatomen; wobei die Alkyl-, Alkoxy-, Alkenyl- und Alkinylgruppen und die aromatischen und heteroaromatischen Ringsysteme in jedem Fall durch einen oder mehrere Reste R⁶ substituiert sein können, und wobei eine oder mehrere Gruppen CH₂ in den Alkyl-, Alkoxy-, Alkenyl- und Alkinylgruppen in jedem Fall durch -R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt werden kann bzw. können;
R⁶, das bei jedem Vorkommen identisch oder unterschiedlich sein kann, ist ausgewählt aus H, D, F, CN, Alkylgruppen mit 1 bis 20 C-Atomen, aromatischen Ringsystemen mit 6 bis 40 C-Atomen oder heteroaromatischen Ringsystemen mit 5 bis 40 aromatischen Ringatomen, wobei die Alkylgruppen, aromatischen Ringsysteme und heteroaromatischen Ringsysteme durch F und CN substituiert sein können.

2. Verbindung nach Anspruch 1, **dadurch gekennzeichnet, dass** Ar¹ eine Phenyl- oder eine Naphthylgruppe ist, die jeweils mit einem oder mehreren Resten R¹ substituiert sein kann.

3. Verbindung nach Anspruch 1 oder 2, **dadurch gekennzeichnet, dass** Ar² ausgewählt ist aus 1-Naphthyl, Fluorenyl, Benzofluorenyl, Indenofluorenyl, Spirobifluorenyl, Benzoindenofluorenyl, Phenanthrenyl, Anthracenyl, Pyrenyl, Benzanthracenyl und Benzophenanthrenyl, die durch einen oder mehrere Reste R² substituiert sein können, oder heteroaromatischen Ringsystemen mit 5 bis 30 aromatischen Ringatomen, die durch Reste R² substituiert sein können.

4. Verbindung nach einem oder mehreren der Ansprüche 1 bis 3, **dadurch gekennzeichnet, dass** Ar² ein heteroaromatisches Ringsystem mit 5 bis 18 aromatischen Ringatomen ist, die durch Reste R² substituiert sein können.

5. Verbindung nach einem der Ansprüche 1 bis 4, **dadurch gekennzeichnet, dass** sie der Formel (I-2) entspricht: 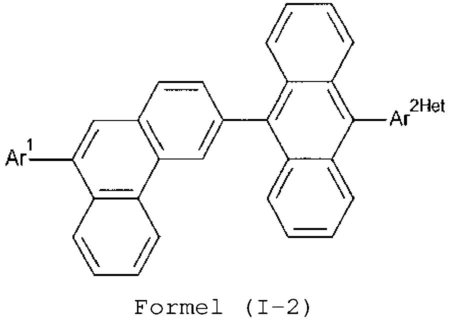 wobei die Phenanthrengruppe in den freien Positionen durch Reste R³ substituiert sein kann, und die Anthracengruppe in den freien Positionen durch Reste R⁴ substituiert sein kann, und
wobei die vorkommenden Variablen wie in Anspruch 1 oder 2 definiert sind, und:
Ar^{2Het} ein heteroaromatisches Ringsystem mit 5 bis 18 aromatischen Ringatomen ist, das durch Reste R² substituiert sein kann.

6. Verbindung nach einem der Ansprüche 1 bis 5, **dadurch gekennzeichnet, dass** R¹, das bei jedem Vorkommen identisch oder unterschiedlich sein kann, ausgewählt ist aus H, D, F, CN, Si(R⁵)₃, geradkettigen Alkyl- oder Alkoxygruppen mit 1 bis 20 C-Atomen oder verzweigten oder cyclischen Alkyl- oder Alkoxygruppen mit 3 bis 20 C-Atomen; wobei die Alkyl- und Alkoxygruppen in jedem Fall durch einen oder mehrere Reste R⁵ substituiert sein können.

7. Verbindung nach einem der Ansprüche 1 bis 6, **dadurch gekennzeichnet, dass** die Reste R³, die bei jedem Vorkommen identisch oder unterschiedlich sein können, ausgewählt sind aus H, D, F, CN, geradkettigen Alkyl- oder Alkoxygruppen mit 1 bis 20 C-Atomen oder verzweigten oder cyclischen Alkyl- oder Alkoxygruppen mit 3 bis 20 C-Atomen, wobei die Alkyl- und Alkoxygruppen in jedem Fall durch einen oder mehrere Reste R⁵ substituiert sein können.

8. Verbindung nach einem der Ansprüche 1 bis 7, **dadurch gekennzeichnet, dass** die Reste R³ H oder D sind.

9. Verfahren zur Herstellung einer Verbindung gemäß Formel (I), **dadurch gekennzeichnet, dass**
- ein arylsubstituiertes Phenanthrenderivat durch eine Sequenz hergestellt wird, die eine übergangsmetallkatalysierte Kopplungsreaktion und eine Carbocyclisierungsreaktion umfasst, und
- dass dieses arylsubstituierte Phenanthrenderivat in einer übergangsmetallkatalysierten Kopplungsreaktion an ein Anthracenderivat gekoppelt wird.

10. Oligomer, Polymer oder Dendrimer, das eine oder mehrere Verbindungen nach einem oder mehreren der Ansprüche 1 bis 8 enthält, wobei die Bindung (en) an das Polymer, Oligomer oder Dendrimer an beliebigen gewünschten Positionen in Formel (I) lokalisiert sein kann bzw. können, die durch R¹, R², R³ oder R⁴ substituiert sind.

11. Formulierung, umfassend mindestens eine Verbindung nach einem oder mehreren der Ansprüche 1 bis 8 oder mindestens ein Oligomer, Polymer oder Dendrimer nach Anspruch 10 und mindestens ein Lösungsmittel.

12. Elektronische Vorrichtung ausgewählt aus der Gruppe bestehend aus organischen integrierten Schaltkreisen (OICs), organischen Feldeffekttransistoren (OFETs), organischen Dünnschichttransistoren (OTFTs), organischen lichtemittierenden Transistoren (OLETs), organischen Solarzellen (OSCs), organischen optischen Detektoren, organischen Photorezeptoren, organischen Feld-Quenchvorrichtungen (OFQDs), organischen lichtemittierenden Zellen (OLECs), organischen Laserdioden (O-Lasern) und organischen Elektrolumineszenzvorrichtungen (OLEDs), umfassend mindestens eine Verbindung nach einem oder mehreren der Ansprüche 1 bis 8 oder mindestens ein Oligomer, Polymer oder Dendrimer nach Anspruch 10.

13. Elektronische Vorrichtung nach Anspruch 12 ausgewählt aus organischen Elektrolumineszenzvorrichtungen, die Anode, Kathode, emittierende Schicht und gegebenenfalls weitere organische Schichten umfassen, **dadurch gekennzeichnet, dass** die mindestens eine Verbindung oder das mindestens eine Oligomer, Polymer oder Dendrimer in einer emittierenden Schicht oder in einer Schicht mit Elektronentransportfunktion vorhanden ist.

14. Organische Elektrolumineszenzvorrichtung nach Anspruch 13, **dadurch gekennzeichnet, dass** die mindestens eine Verbindung oder das mindestens eine Oligomer, Polymer oder Dendrimer als Matrixmaterial in Kombination mit einem Fluoreszenzemitter in einer emittierenden Schicht vorhanden ist, oder als Elektronentransportmaterial in einer Schicht mit Elektronentransportfunktion vorhanden ist.

15. Verwendung einer Verbindung nach einem oder mehreren der Ansprüche 1 bis 8 oder eines Oligomers, Polymers oder Dendrimers nach Anspruch 10 in einer elektronischen Vorrichtung.

## Revendications

1. Composé selon une formule (I) 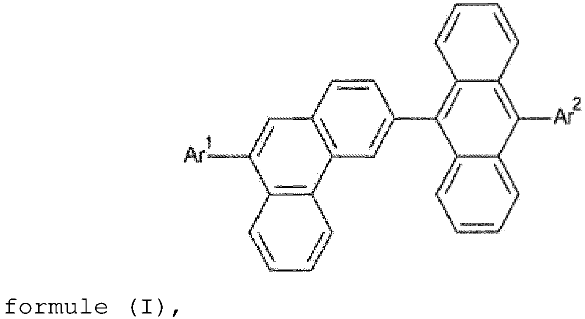 dans laquelle le groupe phénanthrène peut être substitué par des radicaux R³ dans des positions libres, et le groupe anthracène peut être substitué par des radicaux R⁴ dans des positions libres, et dans laquelle ce qui suit s'applique aux variables présentes :
Ar¹ est choisi parmi des groupes aryle possédant 6 à 10 atomes de cycle aromatiques, qui peuvent être substitués par des radicaux R¹, ou des groupes hétéroaryle possédant 5 à 18 atomes de cycle aromatiques, qui peuvent être substitués par des radicaux R¹ ;
Ar² étant choisi parmi 1-naphtyle, qui peut être substitué par des radicaux R², ou des systèmes cycliques aromatiques possédant 13 à 30 atomes de cycle aromatiques, qui peuvent être substitués par des radicaux R², ou des systèmes cycliques hétéroaromatiques possédant 5 à 30 atomes de cycle aromatiques, qui peuvent être substitués par des radicaux R² ;
R¹, R², R⁴ étant choisis, de manière identique ou différente en chaque occurrence, parmi H, D, F, C(=O)R⁵, CN, Si(R⁵)₃, N(R⁵)₂, P(=O)(R⁵)₂, OR⁵, S(=O)R⁵, S(=O)₂R⁵, des groupes alkyle ou alcoxy à chaîne droite possédant 1 à 20 atome(s) de C, des groupes alkyle ou alcoxy ramifiés ou cycliques possédant 3 à 20 atomes de C, des groupes alcényle ou alcynyle possédant 2 à 20 atomes de C, des systèmes cycliques aromatiques possédant 6 à 40 atomes de cycle aromatiques, ou des systèmes cycliques hétéroaromatiques possédant 5 à 40 atomes de cycle aromatiques ; deux radicaux R¹, R² ou R⁴ ou plus pouvant être reliés les uns avec les autres pour former un cycle ; dans lesquels lesdits groups alkyle, alcoxy, alcényle et alcynyle et lesdits systèmes cycliques aromatiques et hétéroaromatiques peuvent être à chaque fois substitués par un ou plusieurs radicaux R⁵, et dans lesquels un ou plusieurs groupes CH₂ dans lesdits groupes alkyle, alcoxy, alcényle et alcynyle peuvent à chaque fois être remplacés par -R⁵C=CR⁵-,-C≡C-, Si(R⁵)₂, C=O, C=NR⁵, -C(=O)O-, -C(=O)NR⁵, NR⁵, P(=O)(R⁵), -O-, -S-, SO ou SO₂ ;
R³ étant choisi, de manière identique ou différente en chaque occurrence, parmi H, D, F, CN, des groupes alkyle ou alcoxy à chaîne droite possédant 1 à 20 atome (s) de C, des groupes alkyle ou alcoxy ramifiés ou cycliques possédant 3 à 20 atomes de C, des groupes alcényle ou alcynyle possédant 2 à 20 atomes de C, deux radicaux R³ ou plus pouvant être reliés les uns avec les autres pour former un cycle ; dans lesquels lesdits groups alkyle, alcoxy, alcényle et alcynyle peuvent être à chaque fois substitués par un ou plusieurs radicaux R⁵, et dans lesquels un ou plusieurs groupes CH₂ dans lesdits groupes alkyle, alcoxy, alcényle et alcynyle peuvent à chaque fois être remplacés par -R⁵C=CR⁵-,-C≡C-, Si(R⁵)₂, C=O, C=NR⁵, -C(=O)O-, -C(=O)NR⁵, NR⁵, P (=O)(R⁵), -O-, -S-, SO ou SO₂ ;
R⁵ étant choisi, de manière identique ou différente en chaque occurrence, parmi H, D, F, C(=O)R⁶, CN, Si(R⁶)₃, N(R⁶)₂, P(=O)(R⁶)₂, OR⁶, S(=O)R⁶, S(=O)₂R⁶, des groupes alkyle ou alcoxy à chaîne droite possédant 1 à 20 atome(s) de C, des groupes alkyle ou alcoxy ramifiés ou cycliques possédant 3 à 20 atomes de C, des groupes alcényle ou alcynyle possédant 2 à 20 atomes de C, des systèmes cycliques aromatiques possédant 6 à 40 atomes de cycle aromatiques, ou des systèmes cycliques hétéroaromatiques possédant 5 à 40 atomes de cycle aromatiques ; dans lesquels lesdits groups alkyle, alcoxy, alcényle et alcynyle et lesdits système cycliques aromatiques et/ou hétéroaromatiques peuvent être à chaque fois substitués par un ou plusieurs radicaux R⁶, et dans lesquels un ou plusieurs groupes CH₂ dans lesdits groupes alkyle, alcoxy, alcényle et alcynyle peuvent à chaque fois être remplacés par -R⁶C=CR⁶-,-C≡C-, Si(R⁶)₂, C=O, C=NR⁶, -C(=O)O-, -C(=O)NR⁶, NR⁶, P(=O)(R⁶), -O-, -S-, SO ou SO₂ ;
R⁶ étant choisi, de manière identique ou différente en chaque occurrence, parmi H, D, F, CN, des groupes alkyle possédant 1 à 20 atome(s) de C, des systèmes cycliques aromatiques possédant 6 à 40 atomes de cycle aromatiques, et des systèmes cycliques hétéroaromatiques possédant 5 à 40 atomes de cycle aromatiques, dans lesquels lesdits groupes alkyle, systèmes cycliques aromatiques et systèmes cycliques hétéroaromatiques peuvent être substitués par F et CN.

2. Composé selon la revendication 1, **caractérisé en ce que** Ar¹ est un phényle ou un groupe naphtyle, qui peuvent chacun être substitués par un ou plusieurs radicaux R¹.

3. Composé selon la revendication 1 ou 2, **caractérisé en ce que** Ar² est choisi parmi 1-naphtyle, fluorényle, benzofluorényle, indénofluorényle, spirobifluorényle, benzoindénofluorényle, phénanthrényle, anthracényle, pyrényle, benzanthracényle, et benzophénanthrényle, qui peuvent être substitués par un ou plusieurs radicaux R², ou des systèmes cycliques hétéroaromatiques possédant 5 à 30 atomes de cycle aromatiques, qui peuvent être substitués par des radicaux R².

4. Composé selon l'une ou plusieurs des revendications 1 à 3, **caractérisé en ce que** Ar² est un système cyclique hétéroaromatique possédant 5 à 18 atomes de cycle aromatiques, qui peuvent être substitués par des radicaux R².

5. Composé selon l'une ou plusieurs des revendications 1 à 4, **caractérisé en ce qu'**il répond à la formule (I-2) 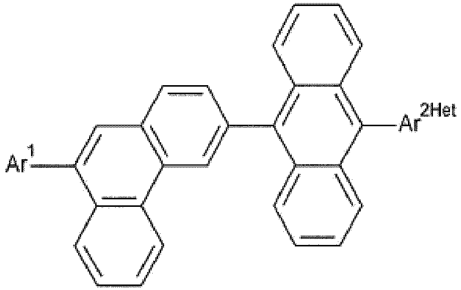 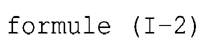 dans laquelle le groupe phénanthrène peut être substitué par des radicaux R³ dans les positions libres, et le groupe anthracène peut être substitué par des radicaux R⁴ dans les positions libres, et
dans laquelle les variables présentes sont définies comme dans la revendication 1 ou 2, et :
Ar^{2Het} est un système cyclique hétéroaromatique possédant 5 à 18 atomes de cycle aromatiques, qui peut être substitué par des radicaux R².

6. Composé selon l'une ou plusieurs des revendications 1 à 5, **caractérisé en ce que** R¹ est choisi, de manière identique ou différente en chaque occurrence, parmi H, D, F, CN Si(R⁵)₃, des groupes alkyle ou alcoxy à chaîne droite possédant 1 à 20 atome (s) de C, et des groupes alkyle ou alcoxy ramifiés ou cycliques possédant 3 à 20 atomes de C ; dans lesquels lesdits groupes alkyle et alcoxy peuvent être à chaque fois substitués par un ou plusieurs radicaux R⁵.

7. Composé selon l'une ou plusieurs des revendications 1 à 6, **caractérisé en ce que** les radicaux R³ sont choisis, de manière identique ou différente en chaque occurrence, parmi H, D, F, CN, des groupes alkyle ou alcoxy à chaîne droite possédant 1 à 20 atome (s) de C, et des groupes alkyle ou alcoxy ramifiés ou cycliques possédant 3 à 20 atomes de C, dans lesquels lesdits groupes alkyle et alcoxy peuvent être à chaque fois substitués par un ou plusieurs radicaux R⁵.

8. Composé selon l'une ou plusieurs des revendications 1 à 7, **caractérisé en ce que** les radicaux R³ sont H ou D.

9. Procédé pour la préparation d'un composé selon la formule (I),
**caractérisé en ce que**
- un dérivé de phénanthrène substitué par aryle est préparé par une séquence comprenant une réaction de couplage catalysée par un métal de transition et une réaction de carbocyclisation, et
- **en ce que** ce dérivé de phénanthrène substitué par aryle est couplé à un dérivé d'anthracène dans une réaction de couplage catalysée par un métal de transition.

10. Oligomère, polymère ou dendrimère contenant un ou plusieurs composés selon l'une ou plusieurs des revendications 1 à 8, dans lesquels la ou les liaison(s) au polymère, à l'oligomère ou au dendrimère peut/peuvent être située(s) au niveau de quelconques positions souhaitées dans la formule (I) qui sont substituées par R¹, R², R³ ou R⁴.

11. Formulation comprenant au moins un composé selon l'une ou plusieurs des revendications 1 à 8 et au moins un oligomère, polymère ou dendrimère selon la revendication 10 et au moins un solvant.

12. Dispositif électronique choisi dans le groupe constitué par les circuits intégrés organiques (CIO), les transistors organiques à effet de champ (TOEC), les transistors organiques à film mince (TOFM), les transistors organiques luminescents (TOL), les cellules solaires organiques (CSO), les détecteurs optiques organiques, les photorécepteurs organiques, les dispositifs organiques d'extinction de champ (DOEC), les cellules électrochimiques luminescentes organiques (CEEO), les diodes laser organiques (O-lasers) et les dispositifs électroluminescents organiques (DELO), comprenant au moins un composé selon l'une ou plusieurs des revendications 1 à 8 au moins un oligomère, polymère ou dendrimère selon la revendication 10.

13. Dispositif électronique selon la revendication 12, choisi parmi des dispositifs électroluminescents organiques, comprenant anode, cathode, couche émettrice et éventuellement des couches organiques supplémentaires, **caractérisé en ce que** l'au moins un composé ou l'au moins un oligomère, polymère ou dendrimère est présent dans une couche émettrice ou dans une couche comportant une fonction de transport d'électrons.

14. Dispositif électroluminescent organique selon la revendication 13, **caractérisé en ce que** l'au moins un composé ou l'au moins un oligomère, polymère ou dendrimère est présent en tant que matériau de matrice en combinaison avec un émetteur fluorescent dans une couche émettrice, ou est présent en tant que matériau de transport d'électrons dans une couche comportant une fonction de transport d'électrons.

15. Utilisation d'un composé selon l'une ou plusieurs des revendications 1 à 8 ou un oligomère, polymère ou dendrimère selon la revendication 10 dans un dispositif électronique.
